# EUROPÄISCHE PATENTSCHRIFT

(11) **EP 3 397 121 B1**
(45) Veröffentlichungstag und Bekanntmachung des Hinweises auf die Patenterteilung: **03.09.2025**
(21) Anmeldenummer: 16828953.6
(22) Anmeldetag: 30.12.2016
(51) Int. Cl.: A47C 20/04, A47C 31/00, A61B 5/00, A47C 27/00

(54) **ANORDNUNG EINES SCHLAF- ODER RUHEMÖBELS UND EINER EXTERNEN KOMPONENTE**
ASSEMBLY OF A SLEEPING OR RESTING FURNITURE AND AN EXTERNAL COMPONENT
AGENCEMENT D'UN MEUBLE DE COUCHAGE OU DE REPOS ET D'UN COMPOSANT EXTERNE

(30) Priorität: 30.12.2015 DE 202015107148 U; 24.05.2016 DE 102016109524; 07.10.2016 DE 202016105634 U
(43) Veröffentlichungstag der Anmeldung: 07.11.2018
(73) Patentinhaber: DewertOkin Technology Group Co., Ltd., Jiaxing, Zhejiang (CN)
(72) Erfinder: GEHRKE, Karsten, 32457 Porta Westfalica (DE); HILLE, Armin, 33659 Bielefeld (DE); LOLEY, Steffen, 49082 Osnabrück (DE); TEWS, Alexander, 33607 Bielefeld (DE)
(74) Vertreter: Kleine, Hubertus
(86) Internationale Anmeldenummer: PCT/EP2016/082919
(87) Internationale Veröffentlichungsnummer: WO 2017/114948

(56) Entgegenhaltungen:
- WO-A1-01/64103
- WO-A1-2013/173640
- JP-A- 2005 177 471
- US-A1- 2008 005 838
- US-A1- 2008 052 837
- US-A1- 2010 101 022
- US-A1- 2011 263 950
- US-A1- 2014 266 733

## Beschreibung

Die Erfindung betrifft eine Anordnung eines Schlaf- oder Ruhemöbels und einer externen Komponente.

Im klinischen Bereich sind Überwachungsgeräte bekannt, die Atmung und/oder Herztätigkeit eines Patienten im Schlaf überwachen, um bei bedenklichen Herzfunktions- und Kreislaufparametern eingreifen zu können.

Inzwischen sind Vorrichtungen zur Überwachung des Schlafzustands anhand von physiologischen Parametern auch für nichtklinische Zwecke im Handel erhältlich. Diese Geräte, die beispielsweise auf einen Nachtisch gestellt werden, erfassen Geräusche und/oder Bewegungszustände während des Schlafs mithilfe von Mikrofonen und/oder Kameras. Aus den erfassten Informationen wird ein Schlafzustand abgeleitet, dessen zeitlicher Verlauf aufgezeichnet wird. Der aufgezeichnete Schlafverlauf kann nachträglich abgerufen und ausgewertet werden. Er kann Aufschluss darüber geben, wie tief und erholsam der Schlaf gewesen ist.

Neben Systemen, die Kamera und/oder Mikrofon einsetzen, ist ein sensorbasiertes System bekannt, bei dem ein druckempfindlicher Sensorstreifen über die Matratze gelegt wird und bei dem dieser Sensorstreifen mit einem Mobiltelefon (Smartphone) verbunden wird, das die Sensordaten aufzeichnet. Aus den Sensordaten werden unter anderem eine Herzfrequenz und eine Atemfrequenz abgeleitet.

Nachteilig an den genannten nichtklinischen Systemen ist eine eingeschränkte Auswertbarkeit der aufgezeichneten Informationen, da die Sensordaten isoliert in dem Mobiltelefon vorliegen.

Die Druckschrift US 2015/0199484 A1 beschreibt ein System zur automatisierten Medikamentendosierung und -ausgabe in einer nichtklinischen Umgebung. Bei der Ermittlung der Medikamentendosierung werden auch gemessene Patientendaten berücksichtigt, unter anderem ein Schlafverhalten. Gemessene Daten werden zur Auswertung an einen Computer übertragen, der nicht notwendigerweise vor Ort in der nicht-klinischen Umgebung aufgestellt sein muss. Weitere relevante Dokumente des Standes der Technik, die zum Verständnis der Erfindung beitragen, werden im Folgenden zitiert: US2008/005838A1, US2008/052837A1, JP2005177471A, WO01/64103A1, US2011/263950A1, US2010/101022A1, WO2013/173640A1 und US 2014/266733A1.

Bei einer umfassenden Überwachung des Schlafzustands auf der Basis von Sensoren, die Vibrationen, Bewegung und/oder Schall messen, fallen umfangreiche Datenmengen an, deren Übertragung an einen externen Computer zur Auswertung eine verfügbare Übertragungsbandbreite übersteigen kann.

Es ist eine Aufgabe der vorliegenden Erfindung, eine Anordnung eines Schlaf- oder Ruhemöbels und einer externen Komponente zu schaffen, die eine umfassende Überwachung des Schlafzustands unter Einbeziehung externer Stellen ermöglichen, ohne auf eine hohe Datenübertragungsbandbreite angewiesen zu sein.

Diese Aufgabe wird erfindungsgemäß durch den Gegenstand des unabhängigen Anspruchs gelöst. Vorteilhafte Ausgestaltungen und Weiterbildungen sind in den abhängigen Ansprüchen angegeben.

Erfindungsgemäß werden somit Sensordaten aus den Sensorsignalen mit einer bestimmten ersten Wiederholrate, auch Abtastrate genannt, erfasst, diese dann lokal ausgewertet, um einen oder mehrere physiologische Parameter zu bestimmen. Dieses erfolgt mit einer zweiten, kleineren Wiederholfrequenz. Es werden dann die ermittelten Werte an die externe Komponente übertragen und nicht die Sensordaten selbst. Durch die lokale Auswertung wird eine deutliche Datenreduktion für die zu übertragenden Daten erzielt. Um die Sensorsignale im Hinblick auf Vibrationen aussagekräftig auswerten zu können, sind Datenraten für die Sensordaten mit Abtastraten im Bereich von Kilohertz sinnvoll. Die daraus ermittelten physiologischen Parameter sind dagegen auch aussagekräftig, wenn nur ein Wert pro Sekunde oder gar pro Minute vorliegt. Durch die lokale Auswertung kann so das zu übertragende Datenvolumen stark verringert werden.

Durch die Datenübertragung an die bzw. einen Datenaustausch mit der externen Komponente können die erfassten und vorausgewerteten Informationen zum einen in einen Kontext mit anderen Informationen gebracht werden, wodurch sich Synergieeffekte nutzen lassen. Zum anderen erleichtert eine Übertragung erfasster und vorausgewerteter Daten an die externe Komponente eine langfristige Speicherung, Archivierung und rechenintensivere Auswertung als es lokal in der Auswerteeinheit möglich ist.

In einer vorteilhaften Ausgestaltung ist die Auswerteeinheit zur Übertragung der Daten an oder zum Datenaustausch mit einem externen Massenspeicher als externe Komponente eingerichtet. Insbesondere kann der externe Massenspeicher eine Cloud sein. Dadurch kann eine hohe Verfügbarkeit und auch lange Speicherdauer der Daten gewährleistet werden. Die Daten können leicht verschiedenen weiteren Instanzen zur Auswertung oder zum Vergleich zur Verfügung gestellt werden. Bei einer derartigen Cloud kann es sich um einen von einem externen Dienstleister angebotenen Speicherplatz handeln, der dezentral und/oder verteilt von Servern bereitgestellt wird, die über das Internet erreichbar sind. Zum anderen kann es sich auch um eine sogenannte persönliche Cloud handeln, bei der ein Speicherort lokal, z. B. in Form eines NAS (Network Attached Storage) - Speichers bereitgestellt wird, der in einem Intranet erreichbar ist. Schließlich wäre auch ein unmittelbar kabelgebunden an die Auswerteinheit angebundener Massenspeicher in diesem Sinne als eine Cloud zu verstehen. Andere Formen einer kabelgebundenen, oder genauer gesagt einer verdrahtungsgebundenen, Cloud umfassen USB-Massenspeichersticks oder Speicherkarten wie SD-Karten.

In einer weiteren vorteilhaften Ausgestaltung ist die Auswerteeinheit zur Übertragung der Daten an oder zum Datenaustausch mit einer Komponente einer Gebäudeautomatisierungsanlage eingerichtet. Die Datenverbindung zwischen der Auswerteeinheit und der Gebäudetechnik ermöglicht es, für die eine oder andere Seite relevante Informationen austauschen zu können, und damit einen Mehrwert für den Benutzer des Systems zu schaffen. Beispielsweise können Informationen der Auswerteinheit, die eine aktuellen Schlafzustand betreffen, an die Gebäudetechnik weitergegeben werden, um beispielsweise Jalousien und/oder Fenster automatisch über die Gebäudetechnik zu öffnen oder zu schließen, ein Raumlicht ein- oder auszuschalten oder ein im Gebäude installiertes Alarmsystem sowohl global als auch alternativ selektiv für nur bestimmte Räume, beispielsweise alle Räume mit Ausnahme des Schlafzimmers, zu aktivieren oder zu deaktivieren. Umgekehrt kann beispielsweise die Auswerteeinheit über die Gebäudetechnik Zugriff auf z.B. ein (Telefon-) Kommunikationssystem oder ein Alarmsystem bekommen, um Alarmmeldungen absetzen zu können, wenn physiologische Parameter in einem bedenklichen Bereich erkannt werden. Unter Gebäudetechnik, auch Gebäudeautomation genannt, sind Einrichtungen zum Erfassen von Umgebungszuständen und Gebäudeparametern (z.B. Temperatur, Beleuchtung, Öffnungszustand von Fenstern oder Türen), zu verstehen, ebenso wie Steuereinrichtungen zum Ansteuern von Gebäudekomponenten (z.B. Lampen, Heizungsanlagen, Lüftungsanlagen, Fenster- oder Türöffner- oder -schließer, Jalousiesteuerungen), die auf Gebäudeparameter wirken. Die Gebäudetechnik kann zumindest teilweise fest installiert sein oder aber, ggf. auch in Teilen, temporär dem Gebäude zugeordnet sein.

Erfindungsgemäß ist die Auswerteeinheit zur Ubertragung der Daten oder zum Datenaustausch mit einer Steuereinrichtung eines elektromotorischen Möbelantriebs eingerichtet. Auf diese Weise können Komponenten der Steuereinrichtung, die bei dem elektromotorischen Möbelantrieb bereits vorhanden sind, auch für die Auswerteeinheit genutzt werden, beispielsweise eine Stromversorgungseinheit, Kommunikationseinrichtungen und/oder ein Gehäuse einschließlich der Anschlussmöglichkeiten. Zudem vereinfacht sich eine Verkabelung des Sensors, wenn die vorhandene Struktur des elektromotorischen Möbelantriebs verwendet wird. Weiter bevorzugt kann die Auswerteeinheit dann die in die Steuereinrichtung des elektromotorischen Möbelantriebs integriert werden. Zudem können an die Steuereinrichtung des elektromotorischen Möbelantriebs gesendete Information unmittelbar von dieser genutzt werden, um geeignete Aktionen des Möbelantriebs anzusteuern. Beispielsweise kann vorgesehen sein, dass bei einem Erkennen bestimmter Schlafzustände, z.B. Atemnot oder Schnarchen, ein Verstellmotor des elektromotorischen Möbelantriebs aktiviert wird, um z.B. ein Rücken- oder Fußteil des Bettes in seiner Position zu verändern, wodurch in der Regel die Person im Bett auch ihre Schlafposition ändert. Weiter kann z.B. vorgesehen sein, eine mit der Steuereinrichtung gekoppelte Beleuchtungseinrichtung, z.B. eine sogenannte Unterbettbeleuchtung, zeitweise einzuschalten, wenn anhand die an die Steuereinrichtung gesendeten Daten anzeigen, dass die Person gerade das Bett verlässt bzw. verlassen hat.

Erfindungsgemäß ist die Auswerteeinheit zur Ubertragung der Daten an oder zum Datenaustausch mit einem Mobilgerät, z.B. einem Mobiltelefon, insbesondere einem Smartphone, oder einem Tablett-Computer, eingerichtet. Hierbei können die Auswerte-, Darstellungs- und auch die Kommunikationsmöglichkeiten des Mobilgeräts vorteilhaft von der Auswerteeinheit genutzt werden.

In einer weiteren vorteilhaften Ausgestaltung sind die von der Auswerteeinheit erfassten physiologischen Parameter beispielsweise eine Herzfrequenz (Pulsfrequenz), eine Atemfrequenz, ein Bewegungsverhalten und/oder ein Schnarchverhalten der Person. Um auch kleine Signale zuverlässig auswerten zu können, weist die Auswerteeinheit vorteilhaft einen Filter, insbesondere einen Tief- oder Bandpassfilter zur Signalverarbeitung auf. Alternativ oder zusätzlich kann auch unmittelbar am Sensor bereits eine erste Signalaufbereitung erfolgen, beispielsweise indem ein Signalverstärker und/oder ein analog und/oder digital arbeitender Signalfilter benachbart zum Sensor angeordnet sind oder in ein Sensorgehäuse integriert sind. Es wird so eine weniger störanfällige Übertragung des Messsignals an die Auswerteeinheit erzielt.

Weiter kann die Auswerteeinheit einen Speicher zum Abspeichern eines Zeitverlaufs der physiologischen Parameter aufweisen. Die Auswerteeinheit kann zudem eine Überwachungseinrichtung zum Vergleichen der physiologischen Parameter mit vorgegebenen Grenzwerten aufweisen, um in einem Fall, in dem eine Gesundheitsgefahr für die Person erkannt wird, diese oder eine weitere Person gewarnt werden kann.

Weiter ist die Auswerteeinheit dazu eingerichtet, Schlafzustände und Zeitanteile der Schlafzustände an einer Schlafperiode aus den Zeitverläufen zu extrahieren. Die Zeitverläufe der physiologischen Parameter und/oder die Schlafzustände und/oder Zeitanteile der Schlafzustände an einer Schlafperiode sind dann bevorzugt Teil der übertragenen Daten.

Bevorzugt weist die Auswerteeinheit eine Übertragungseinheit zur Übertragung der physiologischen Parameter an die externe Komponente auf. Die Übertragungseinheit ist bevorzugt zur drahtlosen Übertragung der physiologischen Parameter an das Mobilgerät eingerichtet, insbesondere über eine WLAN- oder Bluetooth-Übertragungsstrecke. Wenn die physiologischen Parameter an das Mobilgerät übertragen werden, kann ein Vergleich der physiologischen Parameter mit vorgegebenen Grenzwerten auch im Mobilgerät vorgenommen werden.

Die Erfindung wird nachfolgend anhand von Ausführungsbeispielen mithilfe von Figuren näher erläutert. Es zeigen:
- Fig. 1: ein erstes Ausführungsbeispiel eines Schlafmöbels mit einem elektromotorischen Möbelantrieb in einer isometrischen Ansicht;
- Fig. 2: ein zweites Ausführungsbeispiel eines Schlafmöbels mit einem elektromotorischen Möbelantrieb in einem schematischen Blockschaltbild;
- Fig. 3: eine Wiedergabe einer zeitlichen Abhängigkeit von Sensordaten;
- Fig. 4: eine Wiedergabe einer zeitlichen Abhängigkeit physiologischer Parameter; und
- Fig. 5 - 8: verschiedene Szenarien zur Übertragung und Auswertung von Daten an und zum Austausch von Daten mit verschiedenen externen Komponenten in jeweils einer schematischen Skizze.

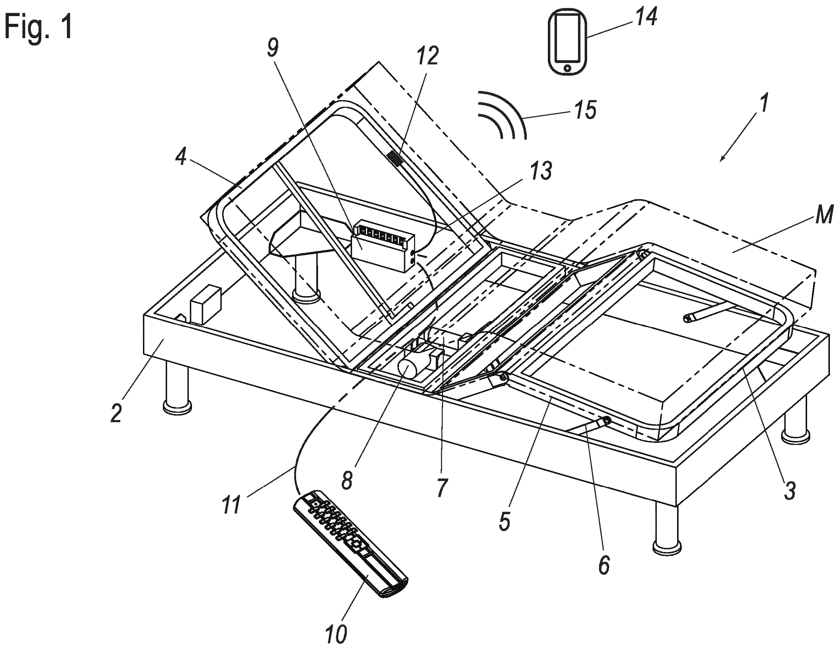

Fig. 1 zeigt ein Bett 1 als Beispiel eines Schlafmöbels mit einem elektromotorischen Möbelantrieb. Das Bett 1 weist wenigstens ein Stützelement 3 zur Aufnahme von z.B. einer Polsterung oder einer Matratze M, auf. Das Bett 1 kann als ein Einzelbett für eine Person oder auch als Doppelbett für mehrere Personen ausgelegt sein. Das Stützelement 3 ist z.B. als ein Lattenrost, als ebene Stützfläche oder dergleichen ausgebildet und an einem Grundelement 2, hier einem Gestell mit Füßen, mit dem das Bett 1 an einem Aufstellort, z.B. Fußboden, aufgestellt ist.

Das Stützelement 3 weist im dargestellten Beispiel ein Rückenteil 4 und ein Beinteil 5 auf, welche relativ zu einem festen mittleren Teil (auch Mittelteil oder Sitzteil genannt) oder relativ zu dem Grundelement 2 beweglich gelagert angeordnet sind. Diese bewegliche Anordnung ist hier mittels eines so genannten Bewegungsbeschlags 6 realisiert. Die Bewegung ist verschiebbar und/oder schwenkbar ausgebildet.

Das beweglich gelagerte Rückenteil 4 und das Beinteil 5 sind jeweils mit einem elektromotorischen Verstellantrieb 7, 8 gekoppelt. So ist das Rückenteil 4 mit dem elektromotorischen Verstellantrieb 7 gekoppelt. Zur Bewegung bzw. Verstellung des Beinteils 5 ist der elektromotorische Verstellantrieb 8 vorgesehen.

Die elektromotorischen Verstellantriebe 7, 8 sind vorliegend als Linearantriebe ausgebildet. Die Linearantriebe weisen einen oder eine Anzahl Elektromotoren auf, wobei jedem Motor ein Drehzahlreduziergetriebe mit wenigstens einer Getriebestufe nachgeschaltet ist. Dem Drehzahlreduziergetriebe kann ein weiteres Getriebe, beispielsweise in Form eines Gewindespindelgetriebes, nachgeschaltet sein, welches aus der Drehbewegung des Motors eine Linearbewegung eines Abtriebsgliedes erzeugt. Das letzte Getriebeglied oder ein damit verbundenes weiteres Glied bildet das Abtriebsglied. Das Abtriebsglied des jeweiligen elektromotorischen Verstellantriebs steht mit dem jeweiligen Möbelbauteil (Rückenteil 4, Beinteil 5) oder alternativ mit einem mit dem Grundelement 2 verbundenes Bauteil in Verbindung, so dass bei einem Betrieb des Elektromotors des jeweiligen Verstellantriebs 7, 8 die beweglichen Möbelbauteile 4, 5 relativ zueinander bzw. relativ zum Grundelement 2 verstellt werden.

Die elektromotorischen Verstellantriebe 7, 8 sind mit einer Steuereinrichtung 9 verbunden. Diese Verbindung kann z.B. als steckbare Kabelverbindung ausgeführt sein, was hier nicht näher dargestellt ist. Die Steuereinrichtung 9 weist eine elektrische Versorgungseinheit auf, welche die elektrische Energie, z.B. aus einem Stromversorgungsnetz, für die elektromotorischen Verstellantriebe 7, 8 bereitstellt. Dazu ist die Steuereinrichtung 9 über ein in diesem Beispiel nicht gezeigtes Netzkabel mit einem Netzstecker mit einem Netzanschluss verbindbar. Der Netzstecker leitet über das Netzkabel die eingangsseitige Netzspannung zu der elektrischen Versorgungseinheit der Steuereinrichtung 9, welche sekundärseitig eine Kleinspannung in Form einer Gleichspannung abgibt.

Alternativ hierzu ist der Steuereinrichtung 9 eine externe netzabhängige Spannungsversorgung mit Netzeingang und mit sekundärseitigem Kleinspannungsausgang vorgeschaltet, welche über die Leitung die Kleinspannung in Form einer Gleichspannung zuführt.

In einer alternativen Ausgestaltung ist die Steuereinrichtung nicht in einem separaten Gehäuse angeordnet, sondern in einen der Verstellantriebe 7, 8 integriert. Dieser Verstellantrieb stellt dann einen Hauptantrieb dar, an den ggf. weitere Verstellantriebe angeschlossen werden können.

In einer weiteren alternativen Ausgestaltung eines elektromotorischen Möbelantriebs kann die Steuereinrichtung verteilt im System angeordnet sein, derart, dass jeder der Verstellantriebe 7, 8 selbst über eine Motorsteuerung verfügt und eine Bus-Kommunikationsschnittstelle aufweist, über die die Verstellantriebe 7, 8 untereinander und mit weiteren Komponenten verbunden sind. Dabei kann vorgesehen sein, dass wenigsten einer der Verstellantriebe 7, 8 ein eigenes Netzteil zu seiner Stromversorgung oder zur Versorgung mehrerer oder aller vorhandenen Verstellantriebe und/oder ggf. weiterer Systemkomponenten aufweist.

Es ist eine Handbedienung 10 vorgesehen, die Bedienelemente aufweist, mit denen die elektromechanischen Verstellantriebe 7, 8 über die Steuereinrichtung 9 steuerbar sind. Die Handbedienung 10 kann in einem Ausführungsbeispiel über ein Kabel mit der Steuereinrichtung 9 verbunden sein. Alternativ kann die Handbedienung 10 mit einer Übertragungseinrichtung für eine drahtlose Übertragung von Signalen zur Steuereinrichtung 9 versehen sein. Die drahtlose Übertragung kann durch eine Funkübertragungsstrecke, eine optische Übertragungsstrecke (z.B. für Infrarotlicht) und/oder eine Ultraschallübertragungsstrecke realisiert sein, wobei die Steuereinrichtung 9 mit einer jeweiligen entsprechenden Empfangseinheit ausgerüstet ist. Weiter alternativ kann die Handbedienung auch die Steuereinrichtung für die Verstellantriebe bilden, z.B. indem der Betriebsstrom der Verstellantriebe direkt über Schalter der Handbedienung geschaltet wird.

Die Bedienelemente können beispielsweise als Taster und/oder Schalter ausgebildet sein. Ferner kann die Handbedienung 10 mit einem Meldeelement, z.B. einer Leuchtiode oder einer Anzeigeeinheit, ausgerüstet. Das Meldeelement dient z.B. zur Funktionsanzeige bzw. Rückmeldung, Fehleranzeige usw.. Ferner kann ein Mobilgerät 14 zur Bedienung der elektromotorischen Verstellantriebe 7, 8 Verwendung finden. Das Mobilgerät 14 kann als Smartphone ausgebildet sein. Die genannten Bedienelemente können als Bereiche eines Touchscreens ausgebildet sein.

Anmeldungsgemäß ist bei dem dargestellten Bett 1 ein Sensor 12 vorgesehen, der Vibrationen, Bewegung und/oder Schall detektiert. Der Sensor 12 ist im dargestellten Ausführungsbeispiel an einem Rahmenbauteil des Rückenteils 4 befestigt. Die Befestigung kann eine Schraub- oder Niet-oder Klebeverbindung sein oder auch eine Rast- oder Klemmverbindung, beispielsweise mithilfe einer Federklemme, die das entsprechende Rahmenbauteil umgreift. Der Sensor 12 ist beispielsweise als piezoelektrisches Bauteil, als elektromagnetisches Bauteil oder als elektromechanisches Bauteil ausgebildet und ist empfindlich für Schwingungen der Unterlage, auf der er befestigt ist, vorliegend also für Schwingungen (Vibrationen) oder Bewegung, die der Rahmen des Rückenteils 4 erfährt. Ein weiterer geeigneter Sensor ist ein elektromechanischer Sensor, z.B. ein mikromechanischer Beschleunigungssensor.

Die genannten Vibrationen umfassen auch Körperschall, der von dem Rückenteil weitergeleitet wird. Unter "Bewegungen" sind insbesondere niederfrequente Schwingungen und Auslenkungen des Sensors 12 zu verstehen, deren Frequenz im Hertz- oder Sub-Hertz-Bereich liegt. Zusätzlich kann der Sensor 12 empfindlich für (Luft-) Schallwellen sein und in diesem Sinne als ein Mikrofon fungieren.

Der Sensor 12 ist über ein Sensorkabel 13 mit der Steuereinrichtung 9 verbunden. Falls benötigt, wird über das Sensorkabel 13 eine Stromversorgung für den Sensor 12 bereitgestellt und es werden vom Sensor 12 ausgegebene Signale an die Steuereinrichtung 9 weitergeleitet. In einer alternativen Ausgestaltung kann der Sensor 12 über eine drahtlose Verbindung, beispielsweise eine Funkverbindung, mit der Steuereinrichtung 9 gekoppelt sein. In dem Fall ist der Sensor 12 mit einer eigenen Energieversorgung, beispielsweise in Form einer gegebenenfalls wiederaufladbaren Batterie versehen.

Bei dem in der Figur 1 gezeigten Bett 1 ist beispielhaft ein einziger Sensor 12 vorhanden. Weiter können auch mehrere Schall- bzw. Schwingungsaufnehmer in einem Sensor oder in verschiedenen Sensoren kombiniert werden, wobei beispielsweise ein piezoelektrisch und ein elektromagnetisch arbeitender Schall- bzw. Schwingungsaufnehmer an gleicher Position oder an verschiedenen Positionen angeordnet sind. Die verschiedenen Sensortypen zeichnen sich durch charakteristische Frequenzbereiche aus, für die sie besonders geeignet sind. Die Kombination verschiedener Sensorarten erlaubt es, ein besonders breites Frequenzspektrum aufnehmen und analysieren zu können.

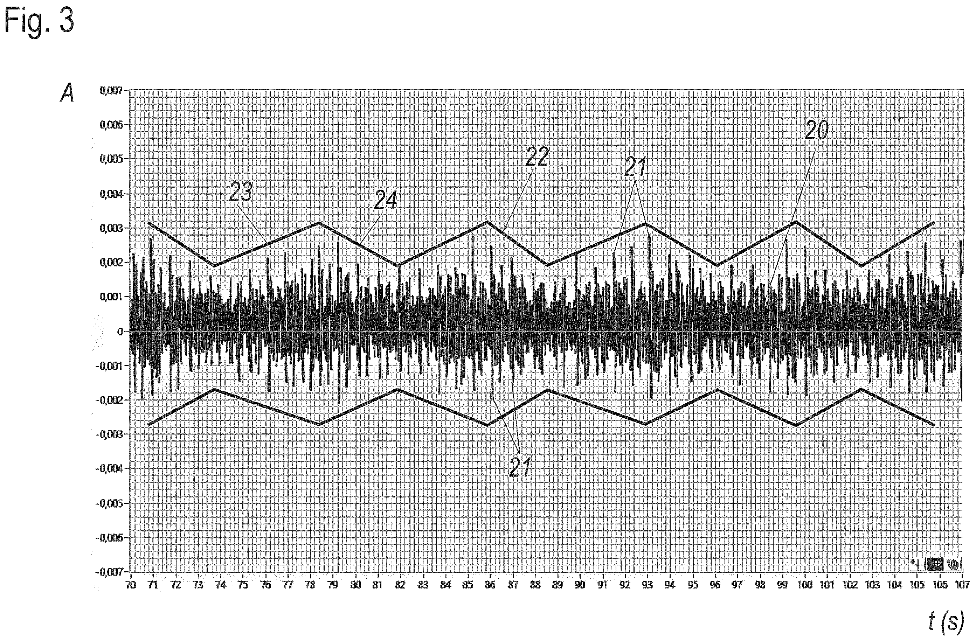

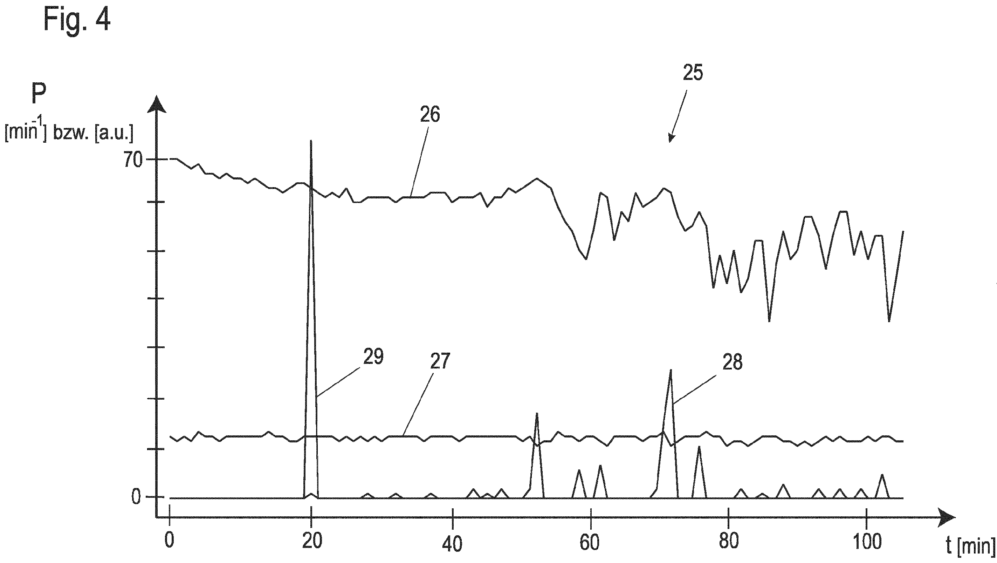

Die Steuereinrichtung 9 umfasst eine Auswerteeinheit zur Verarbeitung und Auswertung der vom Sensor 12 gelieferten Signale. Die Auswerteeinheit umfasst z.B. Verstärker und Filtereinheiten, die es ermöglichen, aus dem vom Sensor 12 übermittelten Signal auf bestimmte Körperfunktionen einer sich im Bett 1 befindenden Person zu schließen. Insbesondere ist die Auswerteeinheit dazu eingerichtet, aus den Signalen des Sensors 12 physiologische Parameter der Person zu ermitteln. Solche Parameter betreffen beispielsweise Herz- und Kreislauffunktionen und umfassen z.B. eine Herzfrequenz und eine Atemfrequenz. Weiter kann ermittelt werden, ob die sich im Bett befindende Person schnarcht. Zudem werden Bewegungen der Person erfasst. Details zur Ermittlung der genannten Parameter aus den Signalen des Sensors 12 werden weiter unten im Zusammenhang mit den Figuren 3 und 4 näher erläutert.

Die bestimmten Parameter werden entweder unmittelbar oder nach Zwischenspeicherung in der Steuereinrichtung 9 in einer direkten Datenübertragung 15 als drahtlose Signale an ein Mobilgerät 14 als externe Komponente übertragen. Das Mobilgerät 14 kann insbesondere ein handelsübliches Mobiltelefon ("Smartphone") oder ein Tablet-Computer sein und ist mit einer entsprechenden Software ("App") ausgestattet, die eine Auswertung und bevorzugt grafische Darstellung der Zeitabhängigkeit der ermittelten Schlafparameter ermöglicht. Als Übertragungsweg für die direkten Datenübertragung kann beispielsweise WLAN (Wireless Lokal Area Network) oder Bluetooth eingesetzt werden.

Alternativ zur direkten Datenübertragung 15 kann gemäß allen Ausführungen eine indirekte Datenübertragung ausschließlich oder zusätzlich vorliegen, wenn der Datenstrom über andere Komponenten geleitet wird und ggf. dort zwischengespeichert wird. Ein Zwischenspeichern kann temporär erfolgen und nach abgeschlossener Datenübertragung kann ein temporärer Zwischenspeicher wieder gelöscht werden, so dass er für eine nachfolgende weitere Zwischenspeicherung wieder zur Verfügung steht. In einer Ausführung kommt die Cloud als Zwischenspeicher in Betracht. Eine indirekte Datenübertragung ist beispielsweise dann gegeben, wenn das Mobilgerät 14 nach einer Art Modem zum Einsatz kommt, die Daten der Auswerteeinheit mittels einer ersten Übertragungsstrecke, beispielsweise in Form einer Bluetooth-Übertragungsstrecke, aufnimmt und mittels einer weiteren Übertragungsstrecke, beispielsweise WLAN oder Mobilfunk, an eine externe Komponente z.B. in Form der Cloud überträgt.

Zudem kann in der Auswerteeinheit der Steuereinrichtung 9 ein Vergleich der gemessenen physiologischen Parameter mit vorgegebenen Grenzwerten für diese Parameter vorgesehen sein. Werden die ermittelten Parameter unmittelbar, das heißt ohne längere Zwischenspeicherung in der Auswerteeinheit, während der Schlafphase an das Mobilgerät 14 übertragen, kann alternativ oder zusätzlich dort ein solcher Vergleich erfolgen. Bei Über- oder Unterschreiten der Grenzwerte bzw. bei einem Verlassen eines oder mehrerer der Parameter aus einem vorgegebenen Bereich ist vorgesehen, dass die Auswerteeinheit bzw. das Mobilgerät 14 ein Alarmsignal ausgibt. Dieses Alarmsignal kann optisch und/oder akustisch unmittelbar von der Auswerteeinheit und damit z.B. der Steuereinrichtung 9 bzw. dem Mobilgerät 14 ausgegeben werden. Alternativ oder zusätzlich kann vorgesehen sein, dass das Mobilgerät 14 eine Alarmmeldung über eine weitere, hier nicht dargestellte drahtlose Übertragungsstrecke (z.B. WLAN, Mobilfunknetz) abgibt. Auf diese Weise kann eine weitere Person benachrichtigt werden, falls sich ungewöhnliche Schlafparameter zeigen. Das dargestellte Bett 1 bzw. der elektromotorische Möbelantrieb mit dem Sensor 12 kann so auch zur klinischen Überwachung bzw. zur Patientenüberwachung oder zur Überwachung von Kleinkindern zum Schutz vor plötzlichem Kindstod eingesetzt werden. Beispielsweise kann eine Alarmmeldung abgegeben werden, wenn eine Person das Bett verlassen hat, ggf. wenn eine Person das Bett seit einer vorbestimmten Zeit verlassen hat oder wenn keine oder nur als kritisch angesehene physiologische Parameter detektiert werden.

Weitere ungewöhnliche Schlafparameter können in Form von Atemaussetzer, Schnarchen, oder sogar Abwesenheiten des zuvor Schlafenden sein. Beim Letztgenannten erfolgt eine An- bzw. eine Abwesenheitskontrolle der Person.

Bei dem dargestellten Ausführungsbeispiel ist der Sensor 12 an einem Rahmenelement des Rückenteils 4 angeordnet. Dieses kann unmittelbar oder über ein Trägerelement erfolgen. Letzteres bietet eine einfachere Nachrüstmöglichkeit des Sensors an ein bereits vorhandenes Bett 1. Andere Anordnungen am Schlafmöbel, also dem dargestellten Bett 1, oder mit ihm verbundener Elemente wie der aufgelegten Matratze M, sind möglich. Der Sensor 12 kann beispielsweise an einem hier nicht sichtbaren Lattenrost montiert sein. Auch eine gegen Verrutschen sichere Montage in oder an der Matratze M ist möglich. Weiter ist die Verwendung mehrerer Sensoren 12 ggf. unterschiedlicher Art, die an gleichen oder unterschiedlichen Stellen im oder am Bett 1 positioniert sind, möglich.

Das Anordnen eines jeden Sensors erfolgt idealerweise in Bereichen des Bettes 1, die benachbart zu den schallerzeugenden Körperteilen der überwachten Person(en) liegen, also z.B. im Herz- / Lungenbereich und im Bereich des Rachens bzw. der Mundöffnung. Denkbare Anbringungsorte sind das Rückenteil 4. Andere Anbringungsorte sind das Sitz- bzw. Mittelteil, welches sich zwischen Rückenteil 4 und Beinteil 5 erstreckt.

Die Verbindung des Sensors 12 mit der Auswerteeinheit der Steuereinrichtung 9, die innerhalb des Betts 1 angeordnet ist, verhindert, dass das Sensorkabel 13 außerhalb des Bettes 1 verlegt werden muss. Die Fixierung des Sensors 12 im oder am Bett 1 stellt eine jederzeit korrekte Positionierung des Sensors 12 und damit eine zuverlässige Auswertung der Daten des Sensors 12 sicher.

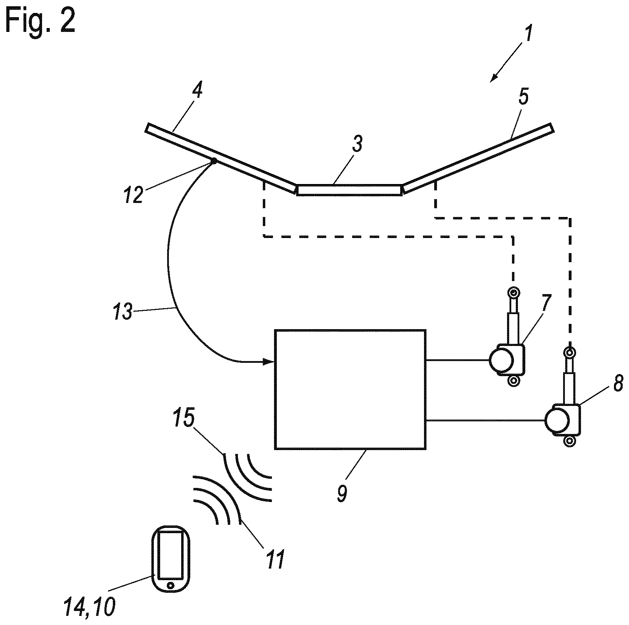

Fig. 2 zeigt ein zweites Ausführungsbeispiel eines Schlafmöbels mit elektromechanischem Möbelantrieb und einem integriertem Sensor 12 in einem schematischen Blockschaltbild. Wiederum ist ein Bett 1 als Beispiel eines Schlafmöbels dargestellt. Gleiche Bezugszeichen kennzeichnen beim Ausführungsbeispiel der Fig. 2 gleiche oder gleich wirkende Elemente wie bei Fig. 1.

In seinem Grundaufbau entspricht der elektromotorische Möbelantrieb gemäß Fig. 2 dem in Fig. 1 gezeigten. Auf die vorstehende Beschreibung wird hiermit verwiesen.

Im Unterschied zum Ausführungsbeispiel der Fig. 1 übernimmt vorliegend das Mobilgerät 14 auch die Funktion der Handbedienung 10. Wiederum ist eine entsprechende Software ("App") für die Funktion als Handbedienung 10 auf dem Mobilgerät installiert.

Die Übertragungsstrecke zwischen dem Mobilgerät 14 und der Auswerteeinheit der Steuereinrichtung 9 ist bidirektional ausgeführt, so dass Steueranweisungen an die Verstellantriebe 7, 8 von dem als Handbedienung genutzten Mobilgerät 14 an die Steuereinrichtung 9 gesendet werden können, und Daten, die den Schlafzustand betreffen, von der Steuereinrichtung 9 an das Mobilgerät 14 übertragen werden können.

Bei den Ausführungsbeispielen der Figuren 1 und 2 ist die Auswerteeinheit für die Signale des Sensors 12 in die Steuereinrichtung 9 integriert. Alternativ ist es möglich, die Auswerteeinheit separat von der Steuereinrichtung 9 in einem eigenen Gehäuse auszubilden. Zur Übertragung der ermittelten physiologischen Parameter kann die Auswerteeinheit dann mit der Steuereinrichtung 9 elektrisch gekoppelt sein, z.B. über eine Datenleitung. Vorteilhafterweise können dann Komponenten der Steuereinrichtung, beispielsweise eine Kommunikationsschnitte zur drahtlosen Datenübertragung, mitgenutzt werden und brauchen nicht zusätzlich auch noch in der Auswerteeinheit vorgesehen sein. Alternativ ist eine Nutzung einer autarken Auswerteeinheit losgelöst von der Steuereinrichtung 9 möglich, insbesondere wenn im Gehäuse der Auswerteeinheit bereits eine Übertragungseinheit zur ggf. drahtlosen Übertragung der ermittelten physiologischen Parameter und/oder vorverarbeiteter Signale des Sensors 12 an eine externe Komponente, z.B. die genannte Cloud oder das Mobiltelefon, vorhanden ist. Die Übertragung kann dabei bidirektional eingerichtet sein.

Ein weiterer Vorteil einer separaten Auswerteinheit ist das Bereitstellen eines einheitlichen Systems für verschiedene Betttypen mit verschiedenen Steuereinrichtungen 9. Somit kann eine erste Auswerteeinheit für Einzelbetten mit einer Vielzahl Varianten und Verstellantrieben 7, 8 Verwendung finden, als auch eine weitere Auswerteeinheit zur Verwendung in Doppelbetten eingesetzt sein.

Fig. 3 zeigt einen Ausschnitt eines gemessenen Signals 20 des Sensors 12 in einem Diagramm. Auf der horizontalen Achse ist der Zeitverlauf t in Sekunden angegeben. Auf der vertikalen Achse ist eine Signalamplitude A in willkürlichen Einheiten dargestellt. Die Signale des Sensors 20 sind dabei mit einer ersten Wiederholfrequenz (Abtastrate) im Bereich von Kilohertz (kHz) aufgenommen bzw. digitalisiert .

Der gezeigte Ausschnitt des Signalverlaufs des Signals 20 ist während einer ruhigen Schlafphase ohne Bewegung und ohne Schnarchen der beobachteten Person. Eine Bewegung der Person äußert sich in Amplituden, die die dargestellte um einen Faktor von einigen 10-100 übersteigen. Bewegungen lassen sich daher sehr leicht identifizieren. Auch ein Schnarchen und die damit einhergehenden Vibrationen sind von dem dargestellten Signalverlauf deutlich unterscheidbar, da sie sich in einer um ein mehrfaches größeren Amplitude widerspiegeln.

In dem in Fig. 3 gezeigten Verlauf des Signals 20 sind regelmäßige Peaks 21 beobachtbar, die vom Herzschlag der Person herrühren und nachfolgend Herzschlag-Peaks 21 genannt werden. Aus dem Abstand der Herzschlag-Peaks 21 kann eine Herzfrequenz ermittelt werden. Der zeitliche Abstand benachbarter Herzschlag-Peaks 21 lässt Aussagen über die Puls-Gleichmäßigkeit zu, die ein Maß für die Tiefe des Schlafes sein kann.

Weiterhin ist in Fig. 3 zu erkennen, dass die Amplitude der Herzschlag-Peaks 21 regelmäßig mit einer geringeren Frequenz variiert. Diese Variation wird durch eine Einhüllende 22 wiedergegeben. Die Einhüllende 22 zeigt sich abwechselnde ansteigende Flanken 23 und abfallende Flanken 24. Der Verlauf der Einhüllenden 22 ist mit der Atmung der Person korreliert. Die ansteigenden Flanken 23 kennzeichnen eine Einatmungsphase und die absteigende Flanke 24 eine Ausatemphase.

Das Beispiel der Fig. 3 zeigt, wie aus den Signalen des Sensors 12 auf Herz-Kreislaufparameter geschlossen werden kann, vorliegend auf Puls- und Atmung. Auf ähnliche Weise können weitere Schlafparameter, wie Bewegungszustände und ein Schnarchen ermittelt werden.

Zur Auswertung der Signale 20 erfolgt eine Filterung der Rohsignale des Sensors 12, insbesondere mittels eines Tiefpassfilters. Auch die Verwendung eines Bandpassfilters mit geeigneten Eckfrequenzen ist möglich. Tiefpass-bzw. Bandpassfilter dienen der Eliminierung von Störfrequenzen. Dabei kann eine signalstärkenabhängige Verstärkung (Automatic Gain Control) erfolgen. Bevorzugt erfolgt die Verarbeitung der Signale mit Hilfe eines digitalen Signalprozessors (DSP). Insbesondere kann vorgesehen sein, die Signale 20 einer Spektralanalyse, z.B. durch eine schnelle Fouriertransformation (FFT-Fast Fourier Transform) zu unterwerfen, um in dem Verlauf der Signale 20 enthaltene Frequenzanteile analysieren zu können. Auch eine Filterung der erhaltenen Spektren kann vorgenommen werden, beispielsweise indem nur Frequenzkomponenten mit einer bestimmten Mindestamplitude weiterverarbeitet werden und andere verworfen werden.

Der Sensor 12 kann zusätzlich oder alternativ auch einer Überwachung der korrekten Funktion des elektromotorischen Antriebs dienen. Eine Betätigung der Verstellantriebe 7, 8 führt zu einer Bewegung der bewegten Möbelteile, beispielsweise des Rückenteils 4 und/oder des Beinteils 5. Zusätzlich resultiert die Betätigung der Verstellantriebe 7, 8 in Vibrationen dieser Möbelteile und auch des gesamten Möbels, die ebenfalls vom Sensor 12 erfasst werden. Diese Vibrationen treten in einem typischen Frequenzbereich auf. Der Signalverlauf spiegelt die Motorbewegung der Verstellantriebe 7, 8 wider. Ein erster typischer relevanter Frequenzbereich liegt im Bereich der Motordrehzahl der Motoren der Verstellantriebe 7, 8. In diesem Frequenzbereich zeigen sich Fehler am Motor selbst oder einem Abtriebszahnrad. Ein weiterer typischer relevanter Frequenzbereich entspricht einem ganzzahligen Bruchteil gemäß einem Übersetzungsverhältnis des Getriebes, das etwa 1:30 bis 1:50 beträgt. In diesem Frequenzbereich deuten sich Fehler in nachgeordneten Getriebestufen oder Wälzlagern an. Ein dritter typischer Frequenzbereich liegt im Bereich von Quietschgeräuschen von Scharnieren, die Teil eines Möbelbeschlags sind. Form und Amplitude sind zum einen typisch für den verwendeten Verstellantrieb 7, 8, zum anderen geben sie Aufschluss über die korrekte Funktion der Verstellantriebe 7, 8 sowie deren Verschleißzustand.

Auch eine Überbelastung einer der Verstellantriebe 7, 8 kann anhand der Signalform der Signale des Sensors 12 erkannt werden. Der Sensor 12 kann somit beispielsweise als Einklemmschutz fungieren, wobei die Steuereinrichtung 9 bei Überbelastung eines der Verstellantriebe 7, 8 diesen Antrieb stoppt bzw. in Gegenrichtung laufen lässt. Auch eine Unterlast am Verstellantrieb 7, 8 kann ein Indiz für ein Einklemmen sein, beispielsweise wenn ein Möbelteil (Rückenteil 4, Beinteil 5) abgelassen wird und der Verstellmotor 7, 8 nahezu kraftlos betrieben wird, deutet dieses auf ein Einklemmen eines Körperteils unter dem sich hinab senkenden bewegten Möbelteil hin. Ein belastungslos betriebener Verstellantrieb 7, 8 ist ebenfalls anhand der Signale des Sensors 12 identifizierbar. Eine Überbelastung kann dadurch gekennzeichnet und sensorisch erfassbar und auswertbar sein, wenn besonders niedrige Frequenzen in einem Bereich typisch für Motorlauffrequenzen und Getriebelauffrequenzen bei gleichzeitiger korrespondierender Amplitude vorliegen.

Fig. 4 zeigt beispielhaft physiologische Parameter P, die aus gemessenen Signalen 20 des Sensors 12, wie beispielsweise in Fig. 3 dargestellt, extrahiert wurden. Anmeldungsgemäß erfolgt dabei eine lokale Auswertung zur Ermittlung der verschiedenen physiologischen Parameter. Beispielsweise kann jeder extrahierte Parameter P einmal pro Minute ermittelt werden. Auf diese Weise wird das große Datenvolumen der digitalisierten Signale 20 des Sensors 12 sinnvoll auf eine deutlich kleinere Datenmenge reduziert. Die Auswertung erfolgt daher bereits in der Steuereinheit 9 bzw. in der in ihr enthaltenen oder auch separat ausgeführten Auswerteeinheit.

Im dargestellten Beispiel betreffen die physiologischen Parameter P eine Pulsfrequenz (Herzfrequenz), eine Atemfrequenz, ein Bewegungsverhalten sowie ein Schnarchverhalten. Es ist jedoch auch denkbar, nur eine Untergruppe dieser beispielhaften physiologischen Parameter P oder auch weitere physiologische Parameter aus den gemessenen Sensordaten 20 zu extrahieren. Im Hinblick auf Details der Extraktion der physiologischen Parameter P wird auf die nachfolgenden Ausführungen verwiesen.

Wie in Fig. 4 dargestellt ist, werden die physiologischen Parameter P als Daten 25 mit einer zweiten Wiederholfrequenz (Datenrate) in Form von Zeitreihen extrahiert und im weiteren Verlauf gespeichert und weiter ausgewertet. In Fig. 4 sind die Daten 25 über einer horizontalen Zeitachse, die eine Zeit t in Minuten dargestellt aufgetragen. Auf der vertikalen Achse sind die physiologischen Parameter P in unterschiedlichen Einheiten wiedergegeben.

Die Pulsfrequenz ist in einer ersten Zeitreihe 26 in der Einheit 1/min (Minuten) dargestellt. Zur Bildung der ersten Zeitreihe 26 wird beispielsweise die Anzahl der Herzschläge aus den Sensordaten 20 bestimmt und über eine Minute gezählt. Der entsprechende Wert bildet einen Datenpunkt in der Zeitreihe 26. Es ist jedoch auch denkbar, über einen kürzeren oder längeren Zeitraum zu ermitteln und beispielsweise zwei Messpunkte pro Minute oder auch nur einen Messpunkte alle 2 oder 5 Minuten bereit zu stellen. Ein größerer zeitlicher Abstand der Datenpunkte in der erste Zeitreihe 26 verringert die Datengröße einer Zeitreihe, führt allerdings zu einer Mittelung, die kurzzeitige physiologische Unregelmäßigkeiten nicht mehr erkennbar werden lässt. Eine minutenweise Erfassung stellt diesbezüglich einen guten Kompromiss aus Datengröße und Aussagekraft der Daten dar. Der Datenumfang der ersten Zeitreihe 26 liegt für eine Zeitlänge von 8 Stunden bei etwa 4 Kilobyte, falls ein Datenpunkt pro Minute in der Zeitreihe 26 gespeichert wird. In jedem Fall ist die Datenrate in der ersten Zeitreihe 26 und den im Folgenden beschriebenen weiteren Zeitreihen dabei deutlich kleiner als die Abtastrate, mit der die Signale 20 des Sensors 12 abgestastet und weiterverarbeitet werden.

Eine zweite Zeitreihe 27 stellt die Atemfrequenz ebenfalls in der Einheit 1/min dar. Auch hier wird vorzugsweise für jede Minute ein Datenpunkt abgespeichert.

Eine dritte Zeitreihe 28 gibt eine aus den Rohdaten 20 extrahierte Bewegung im Schlaf wieder. Diese ist willkürlichen Einheiten angegeben. Gemäß der Abbildung nach Fig. 4 sind zahlreiche Kleinsignale nahe der Nulllinie der dritten Zeitreihe 28 sichtbar, während drei kleine Signale und 2 mittelgroße Signalamplituden 28 als Maß für das Bewegungsverhalten der schlafenden Person stehen.

Eine vierte Zeitreihe 29 schließlich stellt ein aus den Sensordaten 20 extrahiertes Schnarchverhalten ebenfalls im Minutentakt abgespeichert dar. In dem beispielhaften Ausschnitt von etwa 100 Minuten, der in Fig. 4 wiedergegeben ist, ist ein Schnarchen nur einmal bei etwa t = 20 Minuten detektiert worden.

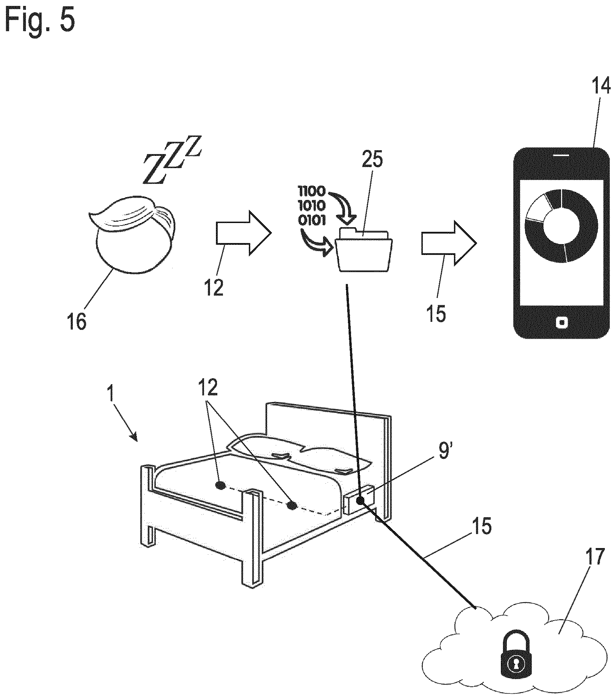

Nachfolgend sind im Zusammenhang mit den Figuren 5 bis 8 verschiedene Szenarien zur Übertragung von Daten an und zum Austausch von Daten mit externen Komponenten dargestellt.

In einem ersten Szenario gemäß Fig. 5 ist beispielhaft ein Doppelbett als Bett 1 dargestellt, das mit hier zwei Sensoren 12 ausgestattet ist, die mit einer Auswerteeinheit verbunden sind. Im Gegensatz zu den in den Figuren 1 und 2 dargestellten Ausführungsbeispielen ist vorliegend eine Auswerteeinheit 9' separat von einer in Fig. 5 nicht dargestellten Steuereinrichtung eines elektromotorischen Möbelantriebs ausgebildet. Es versteht sich, dass die Auswerteeinheit 9' des Ausführungsbeispiels der Fig. 5 auch in eine entsprechende Steuereinrichtung integriert sein kann oder mit der Steuereinrichtung zum Datenaustausch in Verbindung stehen kann. Wie im oberen Teil der Figur angedeutet ist, werden Daten von mindestens einer Person 16 durch den oder die Sensoren 12 erfasst und diese Sensordaten in der Auswerteeinheit 9' ausgewertet, um die Daten 25 zu erhalten. Diese Daten 25, die beispielsweise Zeitreihen 26-29 umfassen, wie sie in Fig. 4 wiedergegeben sind, werden zunächst bevorzugt in der Auswerteeinheit 9' gespeichert.

Die weitere Auswertung der Daten 25 erfolgt auf zwei verschiedenen Wegen, die alternativ oder gleichzeitig verfolgt werden können. Zum einen kann eine unmittelbare Auswertung in Hinblick auf physiologisch von einem Normalwert abweichende und insbesondere bedenkliche Werte der physiologischen Parameter erfolgen. Beispielsweise können, wie schon im Zusammenhang mit den Figuren 1 bis 3 erwähnt ist, Grenzwerte hinterlegt sein, bei deren Über- oder Unterschreitung eine unmittelbare Reaktion erfolgt. Diese Grenzwerte können beispielsweise in der Auswerteeinheit 9' hinterlegt sein, so dass ein Warnsignal unmittelbar von der Auswerteeinheit 9' abgegeben wird, falls anhand der Grenzwerte vom Normalzustand abweichende oder bedenkliche Werte eines oder mehrerer der physiologischen Parameter P detektiert werden.

Ein zweiter möglicher Auswerteweg betrifft Aussagen zum Schlafverhalten, die nicht anhand von Momentanwerten, sondern nur anhand der Zeitreihen (Vgl. Zeitreihen 26 - 29 gemäß Fig. 4) getroffen werden können. Diese Auswertung erfolgt sinnvollerweise erst am Ende einer Schlafperiode. Das Ende einer Schlafperiode wird entweder anhand der physiologischen Parameter P selbst erkannt, beispielsweise wenn über einen vorbestimmten Zeitintervall keine zur Person korrespondierenden Signale mehr vorliegen, oder auch anhand einer manuellen Eingabe, die die Person 16 beispielsweise an einem Bedienelement der Auswerteeinheit 9' vornimmt. Alternativ dazu können auch weitere Informationen, die die Auswerteinheit z. B. über eine Kopplung mit einer Einrichtung der Gebäudeautomatisierung erhält, verwendet werden, um das Ende einer Schlafperiode zu ermitteln, z.B. wenn von der Einrichtung zur Gebäudeautomatisierung mitgeteilt wird, dass eine Kaffeemaschine oder eine Musikanlage eingeschaltet wird. Erfolgt die Erkennung des Endes der Schlafperiode durch die Auswerteeinheit 9' kann umgekehrt die Einrichtung zur Gebäudeautomatisierung über das Ende der Schlafperiode informiert werden und ihrerseits (Küchen-) Geräte wie die genannte Kaffeemaschine oder die Musikanlage einschalten.

Nach dem Ende der Schlafperiode werden die Daten 25 bereits bevorzugt in der Auswerteeinheit 9' auf das Vorliegen bestimmter Schlafzustände, auch Schlafphasen genannt, analysiert. Schlafphasen unterscheiden sich beispielsweise durch die Höhe und/oder Variation des Pulses und/oder der Bewegung innerhalb des Schlafs. Beispielsweise sind anhand von Fig. 4 leicht zwei verschiedene Schlafphasen für t < 50 Minuten und t > 50 Minuten erkennbar. Die erste Schlafphase bei t < 50 Minuten zeichnet sich durch eine nur langsam variierende Herzfrequenz bei gleichzeitig wenig Bewegung im Schlaf aus. Die zweite Schlafphase bei t > 50 Minuten zeigt deutliche Sprünge und Variationen in der Herzfrequenz sowie einen deutlichen Anstieg der Bewegung. Für einen erholsamen Schlaf sind bestimmte der Schlafphasen besonders bedeutend. In einer Auswertung der Daten 25 wird das Vorliegen der verschiedenen Schlafphasen erkannt und ihr Anteil an der gesamten Schlafdauer ermittelt. Die so ausgewerteten Daten 25 erlauben es, die Schlafqualität in einer Größe, beispielsweise als Zahlenwert von 0 bis 100 zusammen zu fassen. Die Informationen über Schlafphasen und Anteile der Schlafphasen an der gesamten Schlafdauer bzw. die Schlafqualität kann beispielsweise als ein Kopf ("Header") den Daten 25 vorangestellt werden.

Unter den jeweiligen Schlafphasen, welche in der Literatur auch als Schlafstadien bezeichnet werden, versteht sich die Einteilung des Schlafes gemäß verschiedener Intensität. Aus der Literatur sind die Schlafphasen Leichtschlaf (N1), Schlaf (N2) und ausgiebiger Tiefschlaf (N3) sowie REM (Rapid Eye Movement)-Schlaf (R) bekannt. Die anmeldungsgemäß genannten Schlafphasen sollen beispielsweise auf vier Schlafphasen begrenzt sein. Bei anderen Ausführungen zur Aufteilung und Zuordnung der Daten 25 zur jeweiligen Schlafphase können wenigstens zwei Schlafphasen Verwendung finden. Als Alternative kann auch aus dem Umfang der zur Verfügung stehenden Daten die Anzahl der Schlafphasen anpassbar ausgebildet sein, wenn beispielsweise durch eine sehr kurze Schlafdauer eine begrenzte Datenmenge zur Auswertung durch die Auswerteeinheit 9' zur Verfügung steht.

Der wenigstens eine Sensor 12 bzw. das wenigstens eine Sensorsignal läßt also Rückschlüsse auf die jeweilige Schlafphase zu. Die vom Sensor 12 bzw. die vom Sensorsignal ableitbaren und zur Verfügung gestellten Daten 25 unterscheiden sich von den gewonnenen Informationen aus einer Polysomnographie, da der wenigstens eine anmeldungsgemäße Sensor 12 keine Gehirnaktivitäten erfassen kann. Aber es hat sich gezeigt, dass durch Erfassen der anmeldungsgemäß genannten physiologischen Parameter durch den wenigstens einen Sensor 12 Signale, Daten 25 und Informationen bereitstellt sind, welche gemäß einer nachgeschalteten Aufbereitung und Filterung und Auswertung Rückschlüsse auf die jeweilige Schlafphase zulässt. Als Berechnungsresultat ist es somit auch möglich, einen einzigen Zahlenwert kennzeichnend für den getätigten Schlaf auszugeben. Hierzu ist es dann zweckdienlich, wenn jeder Schlafphase eine kalkulatorische Gewichtung zugeordnet ist. Alternative Berechnungsresultate können Schlafphasen vereinzelt zusammenfassen.

Es hat sich herausgestellt, dass in Abhängigkeit der jeweiligen Schlafphase sich der jeweilige Verlauf wenigstens einer Zeitreihe charakteristisch ändert. Fig. 4 illustriert die Änderung des charakteristischen Verlaufes der ersten Zeitreihe 26 entlang der Abszisse, wobei eine signifikante Änderung des Signalverlaufes erkennbar ist. Während in der linken Bildhälfte das Signal einen nahezu gleichmäßig monoton fallenden Verlauf einnimmt, so ist der Signalverlauf in der rechten Bildhälfte von starken Schwankungen geprägt. Durch eine rechnergestützte Auswertung des jeweiligen Signalverlaufes der jeweiligen Zeitreihe 26, 27, 28, 29 können so verschiedene Schlafphasen erkannt werden. So können beispielweise statistische Auswertungen hilfreich sein, wobei die Höhe eines statistischen Berechnungsmaßes ein Maß zur Bestimmung einer Schlafphase und sogar einer Schlafgüte herangezogen oder dienen kann. Beispielsweise ist die Standardabweichung als statistisches Berechnungsmaß des Signalverlaufes der ersten Zeitreihe 26 in der linken Bildhälfte deutlich kleiner als die Standardabweichung des Signalverlaufes der ersten Zeitreihe 26 der rechten Bildhälfte.

Die rechnergestützte Auswertung umfasst ferner eine logische Verknüpfung von Bedingungen und Schwellwerten und kann einzig auf eine jeweilige Zeitreihe 26, 27, 28, 29 Anwendung finden oder - wie hier beispielhaft anhand der Zeitreihe 26 vorgestellt - im Anschluss an die zuvor genannte statistische Auswertung erfolgen. Als Resultat des wenigstens einen statistischen Berechnungsmaßes können aus deren Höhe verschiedene Aussagen zugewiesen werden.

Ein statistisches Berechnungsmaß geringer Höhe kann einer ersten Aussage zugewiesen werden. Ein statistisches Berechnungsmaß mit einer Höhe innerhalb einer vorgegebenen Spanne kann einer weiteren Aussage zugewiesen werden. Ein statistisches Berechnungsmaß in einer vorgegebenen Höhe bei gleichzeitig vorliegender Aussage selbiger einer anderen Zeitreihe kann wiederrum einer weiteren Aussage zugewiesen werden. Jede Aussage ist als rechnerischer Zahlenwert abbildbar. Jede Aussage oder die rechnerische Zusammenschau ausgewählter Aussagen steht für das Abbild der jeweiligen vorliegenden Schlafphase und/oder für das Abbild des jeweiligen vorliegenden Schlafqualitätswerts. Auf Basis dieser Verfahren erfolgt das zuvor genannte Extrahieren der physiologischen Parameter P aus den Signalen des Sensors 12 bzw. aus den Daten, die aus diesen Signalen des Sensors 12 erzeugt werden.

Als statistische Berechnungsmaße und/oder dessen Hilfsmaße kommen die bekannten Maße wie beispielsweise Standardabweichung, Mittelwert, Varianz, Intervallgrenzen, Normalverteilung, Differenzierung nach der Zeit, Variationskoeffizient, und dergleichen in Frage.

Bei dem in Fig. 5 dargestellten Ausführungsbeispiel werden nach Beendigung des Schlafs die zusammengefassten Schlafdaten, d. h. die Anteile der verschiedenen Schlafphasen und der daraus ermittelte Schlafqualitätswert über eine direkte Datenübertragung 15, bevorzugt eine drahtlose Datenübertragung über Bluetooth oder WLAN, an das Mobilgerät 14 übertragen. Diese Informationen können nach Beendigung des Schlafs damit der Person 16 entsprechend aufbereitet dargestellt werden. In einer alternativen Ausgestaltung ist es denkbar, auch die Daten 25 selbst an das Mobilgerät 14 als externe Komponente zu übertragen, woraufhin eine Auswertung der Schlafphasen im Mobilgerät 14 erfolgt.

Um auch eine langfristigere Analyse und Kontrolle des Schlafverhaltens zu ermöglichen, kann zusätzlich vorgesehen sein, über einen entsprechenden Datenstrom 15 die Daten 25 und/oder die daraus ermittelten Informationen über die Schlafphasen an einen externen Speicherort, eine so genannte Cloud 17 als weitere externe Komponente zu übertragen. Da es sich um ggf. sensible persönliche Daten handelt, erfolgt eine derartige Übertragung und Speicherung gesichert, bevorzugt verschlüsselt. Die Daten können in der Cloud 17 aufgrund einer größeren Speicherkapazität für einen längeren Zeitraum gespeichert werde, als es in der Auswerteeinheit 9' selbst sinnvoll ist.

Ferner sind unterschiedliche Arten von Daten 25 zur Ablage in der Cloud 17 vorgesehen. Auf persönliche Daten einer Person 16, darunter fallen auch die physiologischen Daten, genießt ausschließlich die Person 16 selbst Schreib- und Leserechte. Systemspezifische Daten 25, welche beispielsweise Auskünfte über den jeweiligen Status eines Verstellantriebs 7, 8 oder der Steuereinrichtung 9 oder der Auswerteeinheit 9' betreffen, sowie Daten korrespondierend zum Verschleiß dieser Komponenten oder Daten korrespondierend zum Verschleiß des Bettes 1 oder zum Verschleiß einer Matratze, können ebenfalls in der Cloud 17 abgelegt oder zumindest zwischengespeichert werden. Im Servicefall hat der Servicecenter 18 somit die Möglichkeit, ausgewählte Daten zum Zwecke der Ferndiagnose einzusehen.

Ferner besteht die Möglichkeit, einer statistischen Auswertung eine Auswahl zuvor systemspezifischer Daten 25 zuzuführen.

Im Rahmen der vorliegenden Anmeldung ist dabei der Begriff "Cloud" weit zu fassen. Zum einen kann es sich um einen von einem externen Dienstleister angebotenen Speicherplatz der dezentral und/oder verteilt von Servern bereitgestellt wird, die über das Internet erreichbar sind. Zum anderen kann es sich auch um eine sogenannte persönliche Cloud handeln, bei der ein Speicherort lokal, z. B. in Form eines NAS (Network Attached Storage) - Speichers bereitgestellt wird, der in einem Intranet erreichbar ist. Schließlich wäre auch ein unmittelbar kabelgebunden an die Auswerteinheit angebundener Massenspeicher in diesem Sinne als eine Cloud zu verstehen. Andere Formen einer kabelgebundenen, oder genauer gesagt einer verdrahtungsgebundenen, Cloud umfassen USB-Massenspeichersticks oder Speicherkarten wie SD-Karten. Diese Cloud-bildenden Speicherelemente können an verschiedenen Orten und Komponenten vorgesehen sein. Als beispielhafte Orte sollen Smartphone, Auswerteeinheit 9', Steuereinrichtung 9, Bett 1, Speicherbaustein der Gebäudetechnik 19, etc. genannt sein.

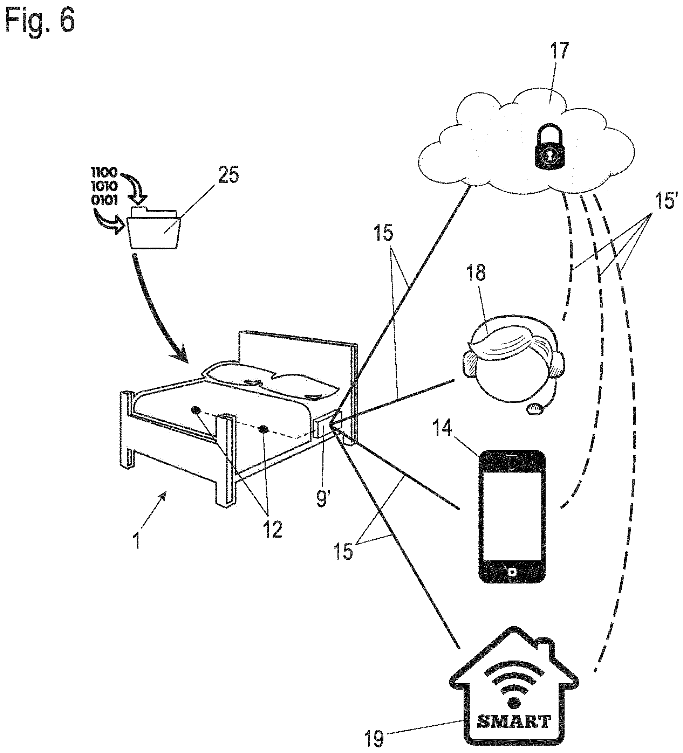

Fig. 6 zeigt ein weiteres Ausführungsbeispiel einer Auswerteeinheit 9' für Sensorsignale eines Sensors 12 mit gegenüber dem Beispiel von Fig. 5 erweiterten Möglichkeiten zur Datenspeicherung, -übertragung und -auswertung.

Grundsätzlich entspricht das in Fig. 6 gezeigte System das dem in Fig. 5 gezeigten auf dessen Beschreibung hiermit verwiesen wird. Beim Ausführungsbeispiel der Fig. 5 ist die Auswerteinheit zur Datenübertragung an das Mobilgerät 14 und die Cloud 17 eingerichtet. Beim Ausführungsbeispiel der Fig. 6 ist die Auswerteeinheit 9' zusätzlich dazu eingerichtet, einen Datenaustausch über eine direkte Datenstrecke 15 auch mit einem Servicecenter 18 sowie Einrichtungen einer lokalen Gebäudeautomationstechnik 19, nachfolgend abkürzend Gebäudetechnik 19 genannt, eingerichtet. Das Servicecenter 18 und die Gebäudetechnik 19 stellen in dem Sinne neben dem Mobilgerät 14 und der Cloud 17 weitere externe Komponenten dar.

Die Verbindung zu sowohl dem Mobilteil 14, als auch dem Servicecenter 18 oder der Gebäudetechnik 19 kann dabei unmittelbar von der Auswerteeinheit 9' erfolgen. Es ist alternativ oder zusätzlich denkbar, dass Daten vorrangig von der Auswerteeinheit 9' über die direkte Datenverbindung 15 in die Cloud 17 zu übertragen. Sowohl das Servicecenter, als auch das Mobilgerät 14 und die Gebäudetechnik 19 können die Daten alternativ dann aus der Cloud 17 beziehen, was im Hinblick auf die Auswerteeinheit 9' einer indirekten Datenübertragung 15' entspricht. Dabei können, wie nachfolgend noch näher erläutert wird, individuelle Zugriffsrechte auf bestimmte der Daten den bestimmten Datenabnehmern zugewiesen sein.

Das Servicecenter 18 dient der Überprüfung der technischen Funktionalität der Auswerteeinheit 9' und ggf. einer Steuereinrichtung 9 eines mit der Auswerteeinheit 9' oder mit dem Bett 1 verbundenen elektromotorischen Möbelantriebs. Wie zuvor erwähnt, kann die Auswerteeinheit 9' auch in eine derartige Steuereinrichtung 9 integriert sein oder mit dieser in Verbindung stehen.

Die Steuereinrichtung 9 kann ebenfalls als eine externe Komponente angesehen werden, mit der die Auswerteeinheit 9' Daten austauscht. Insbesondere kann die über die Auswerteeinheit 9' Informationen über den aktuellen Betriebszustand des elektromotorischen Möbelantriebs empfangen und ebenfalls in Form von Zeitreihe ablegen und z.B. an die Cloud 17 weiterleiten. Es ist auch möglich, derartige Zeitreihen, die den elektromotorischen Möbelantrieb betreffen, den Daten 25 hinzuzufügen.

Das Servicecenter 18 kann, insbesondere auf eine Reklamationsanfrage eines Benutzers des Systems hin, auf in der Auswerteeinheit 9' bzw. in der Cloud 17 gespeicherte Daten zugreifen, die Aufschluss über die Funktionalität der Auswerteeinheit 9', angeschlossener Sensoren 12 oder Komponenten des elektromotorischen Möbelantriebs erhalten. Dazu gehören beispielsweise auch Informationen über die Daten 25, beispielsweise ob und für welche Tage die Daten 25 vorliegen. Zum Schutz der Persönlichkeit des Benutzers werden dagegen bestimmte Inhalte der Daten 25 selbst, insbesondere die in den Daten 25 hinterlegten Zeitreihen 26-19 der physiologischen Parameter P, dem Servicecenter 18 nicht zur Verfügung gestellt. Weiterhin ist des Servicecenter 18 in der Lage, einen Funktionscheck, eine Fehleranalyse, einen Softwaretest, ein Softwareupdate oder dergleichen bei dem Bett 1 und deren Steuereinrichtung 9 bzw. Auswerteeinheit 9' vorzunehmen. Auf Basis solcher Maßnahmen und der Ferndiagnose ist es möglich, ohne einen Servicetechniker mögliche Fehler zu beheben oder auszuschließen. Ferner ist es möglich, einen Fehler näher lokalisieren zu können, so dass ein Servicetechniker seine zielgerichteten Leistungen erbringen kann.

Prinzipiell ist im Rahmen einer Ferndiagnose eine Onlineverbindung zwischen dem Bett 1 und dem Servicecenter 18 vorgesehen. Dabei kann es zweckdienlich sein, wenn das Servicecenter 18 über einen eingeschränkten Zugriff auf die Steuereinrichtung 9 und auf die Auswerteeinheit 9' verfügt. Das Servicecenter 18 verfügt über die Möglichkeit, alle Informationen über den regulären Betrieb der Auswerteeinheit 9' und/oder der Steuereinrichtung 9 abrufen zu können. Somit können Funktionsdiagnosen durchgeführt werden. Ferner ist es zweckdienlich, wenn der Servicecenter 18 Funktionstests durchführen kann, indem der Auswerteeinheit 9' und/oder der Steuereinrichtung 9 Steuerbefehle zum Ausführen von Aktionen übermittelt werden. Aus Sicherheitsgründen kann dies nur im Beisein und mit Einwilligung des Benutzers bzw. der Person 16 direkt in unmittelbarer Nähe des Bettes 1 erfolgen. Als Einwilligung oder Sicherheitsabfrage oder Sicherheitsbedingung kann durch das Bestätigen des Benutzers oder der Person 16 durch das Betätigen einer Taste an dem Bett 1, an der Steuereinrichtung 9, an der Auswerteeinheit 9' und/oder an dem Mobilgerät 14 bzw. einer Handbedienung erfolgen, welche mit der Steuereinrichtung 9 in Verbindung steht. Alternativ kann die Sicherheitsabfrage bzw. Einwilligung durch die Übermittlung der Rufnummer des Mobilgeräts 14 erfolgen, wenn die Person 16 in der Registrierdatenbank des Servicecenters 18 gelistet ist.

Unter den Begriff Ausführen von Aktionen fällt auch das Zurücksetzen, Kalibrieren oder Justieren von Sensoren 12 und/oder der Auswerteeinheit 9'. Somit lassen sich Einflüsse auf gemessene oder detektierte Werte des jeweiligen Sensors 12 begründet durch einen mechanischen Verschleißes des Bettes 1 ausgleichen.

Es ist also auf einfachste und bequemste Art und Weise ein vollumfänglicher Funktionstest aller Hard- und Software des Bettes 1 auch aus der Ferne möglich, sowie eine Justierung oder Kalibrierung des wenigstens einen Sensors 12 und/oder der Auswerteeinheit 9' vornehmen zu können.

In einer weiteren Ausgestaltung dient das Mobilgerät 14 als Kommunikationsverbindungsgerät nach Art eines Modems. Ein Kommunikationsverbindungsgerät, im Folgenden abgekürzt als Modem bezeichnet, stellt die Verbindung zwischen Bett 1, Steuereinrichtung 9 und/oder Auswerteeinheit 9' auf der einen Seite und dem Mobilfunknetz auf der anderen Seite her. Somit ist es auf einfachste Weise möglich, die Verbindung zwischen dem Bett 1, der Steuereinrichtung 9 und/oder der Auswerteeinheit 9' mit dem Servicecenter 18 herstellen zu können. Idealerweise kommuniziert das Bett 1, die Steuereinrichtung 9 und/oder die Auswerteeinheit 9' über eine erste Funkübertragungsstrecke mit dem Mobilgerät 14. Die erste Übertragungsstrecke ist als Bluetooth-Übertragungsstrecke oder als WLAN- Übertragungsstrecke ausgebildet. Die zweite Übertragungsstrecke ist ebenfalls als Funkübertragungsstrecke ausgebildet und zur Kopplung über dem Mobilfunknetz mit dem Internet vorgesehen.

Im regulären Betriebszustand ist der Austausch von Daten und Informationen zwischen der ersten und der zweiten Übertragungsstrecke deaktiviert und kann ausschließlich durch die Person 16 zur Kontaktaufnahme mit einem Servicecenter 18 aktiviert werden. Zur Aktivierung des Daten- und Informationsaustausches kann eine Passwortabfrage dienlich sein oder die Eingabe einer Seriennummer beispielsweise der Seriennummer der Steuereinrichtung 9. Ferner kann eine Verschlüsselung der zweiten Übertragungsstrecke bzw. eine Verschlüsselung der zu übertragenen Daten und Informationen vorgesehen sein. Auf Basis dieser Sicherheitsmaßnahmen ist ein Maximum an Betriebssicherheit für die Person 16 gegeben.

Ist kein Mobilfunknetz vorhanden, so kann an dessen Stelle ein Telefonnetz oder ein WLAN-Netz oder ein LAN-Netz heran gezogen werden, wobei die eingangs genannten ersten und zweiten Übertragungsstrecken an die jeweilige Peripherie angepasst ist und als verdrahtete Übertragungsstrecke ausgebildet sein kann. So ist im einfachsten Fall möglich, dass die Steuereinrichtung 9 und/oder die Auswerteeinheit 9' über einen Telefonanschluss verfügen und die eingangs genannten Übertragungsstrecken zu einer Übertragungsstrecke in verdrahteter Form zusammen fallen. Alternativ ist auch vorgesehen, die erste Übertragungsstrecke als LAN-Übertragungsstrecke (Local Area Network) auszubilden oder ferner alternativ als WLAN-Übertragungstrecke (Wireless Local Area Network) auszubilden.

Unter der Gebäudetechnik 19 versteht man üblicherweise ein System, das einen Status von zumeist elektrischen (z. B. Licht, Alarmsystem), mechanischen (Fenster, Jalousien) oder wärmetechnischen (Heizung, Klimaeinrichtung) Installationen erfassen und/oder steuern kann. Die Datenverbindung 15 zwischen der Auswerteeinheit 9' und der Gebäudetechnik 19 ermöglicht es, für die eine oder andere Seite relevante Informationen austauschen zu können, und damit einen Mehrwert für den Benutzer des Systems zu schaffen. Beispielsweise können Informationen der Auswerteinheit , die eine aktuellen Schlafzustand betreffen, an die Gebäudetechnik 19 weitergegeben werden, um beispielsweise Jalousien und/oder Fenster automatisch über die Gebäudetechnik 19 zu öffnen oder zu schließen, ein Raumlicht ein- oder auszuschalten oder ein im Gebäude installiertes Alarmsystem sowohl global als auch alternativ selektiv für nur bestimmte Räume, beispielsweise alle Räume mit Ausnahme des Schlafzimmers, zu aktivieren oder zu deaktivieren. Ein Aufstehen aus dem Bett 1 während der Nacht kann beispielsweise von der Auswerteeinheit 9' erfasst und an die Gebäudetechnik 19 weitergegeben werden, um automatisch ein Flurlicht z. B. für einen Toilettengang einzuschalten. Ferner können dabei die Alarmsysteme des Gebäudes selektiv deaktiviert sein.

Die Auswerteeinheit 9' erkennt, dass die jeweilige Person aus dem Bett 1 aufgestanden ist, weil bestimmte oder alle Signale des wenigstens einen Sensors 12 fehlen. Hierzu kann es vorteilhaft sein, weitere Sensoren 12 vorzusehen. Als weiterer Sensor 12 kann ein kraftsensitiver Sensor 12 nützlich sein. Indem sich bestimmte Signale von Sensoren 12 verändern oder nicht mehr vorhanden sind, berechnet die Auswerteeinheit 9' die logische Schlussfolgerung, dass die jeweilige Person aus dem Bett aufgestanden ist. Sodann gibt die Auswerteeinheit 9' ein Signal aus zur Einleitung von Aktionen wie beispielsweise einem Schalten von Beleuchtungen in Form von Unterbettbeleuchtungen oder dem eingangs genannten Flurlicht. Das Ausführen der Aktion Schalten der Unterbettbeleuchtung erfolgt durch die Steuereinrichtung 9. Das Ausführen der Aktion Schalten des Flurlichts erfolgt durch die Gebäudetechnik 19. Das Ausführen jeder Aktion kann noch ereignisorientiert sein, beispielsweise in Abhängigkeit der Tageszeit oder der Helligkeit. Schlußendlich erfolgt durch die Auswerteeinheit 9' die Abgabe und Übergabe von Informationen, korrespondierend zum Status der jeweiligen Person 16 im oder nicht im Bett 1.

Jede Art von logischen Schlußfolgerungen oder Informationen kann von der Auswerteeinheit 9' und/oder von der Steuereinrichtung 9 entlang einer drahtgebunden oder drahtlosen Übertragungsstrecke abgesendet werden. Empfangen werden diese Informationen beispielsweise von der Gebäudetechnik 19 oder von einem Mobilgerät 14.

Das Erfassen, ob eine Person 16 sich im Bett befindet oder nicht, kann auf vielfältige Weise genutzt werden. Somit ist es beispielsweise für die häusliche Pflege sehr hilfreich, wenn diese über den Zustand der Person 16 im Bett in Kenntnis versetzt ist und die Information darüber über eine der genannten Übertragungsstrecken erhält.

Darüber hinaus kann es auch für Hotelpersonal oder dessen Arbeitsvorbereitungen sehr hilfreich sein, zu wissen, ob eine Person 16 sich noch im Bett befindet oder nicht. Somit können zielgerichtet Weckdienste oder Zimmerservices eingerichtet werden. Über die Gebäudetechnik 19 kann auch ein Zugriff auf eine Kommunikationsinfrastruktur, beispielsweise ein Telefonnetz erfolgen, so dass ggf. ein automatischer Anruf abgesetzt werden kann, wenn von der Auswerteeinheit 9' bestimmte und bedenkliche physiologische Zustände erkannt werden.

Auch Informationen über ein Raumklima und/oder ein Umgebungsklima (Wetter, Tageslicht) werden häufig von der Gebäudetechnik 19 erfasst. Solche Informationen kann die Auswerteeinheit 9' ebenfalls entgegennehmen und z.B. wiederum in Form von Zeitreihen zwischenspeichern und ggf. als Teil der Daten 25 oder separat davon in der Cloud 17 langfristiger ablegen. Derartige Daten können nachträglich in aufwändigeren Vergleichsuntersuchungen eingesetzt werden, um Störeinflüsse auf das Schlafverhalten des Benutzers des Systems aufzudecken. Daten der Gebäudetechnik 19 können beispielsweise mit dem verschiedenen Schlafphasen oder einem Wechsel zwischen den Schlafphasen in Verbindung gebracht werden.

Neben der Erfassung, Speicherung, Auswertung und Abbildung von physiologischen Parametern und Transformierung dieser zu weiteren Rechenergebnissen in Form eines Schlafqualitätswertes können somit auch andere Parameter der Umwelt betreffend zur Erfassung, Speicherung und Auswertung heran gezogen werden, um den Schlafqualitätswert noch spezifischer bestimmen zu können. Denn als physiologisch besonders unangenehm empfundene Parameter wie beispielsweise eine zu warme, zu kalte oder zu helle Umgebung während der Ruhephase beeinträchtigen die Schlafqualität.

Es ist somit alternativ vorgesehen, diese umweltbezogenen Parameter wie Temperatur, Luftfeuchte, Grundgeräusche, Helligkeit oder dergleichen separat und parallel zu den physiologischen Parametern zu erfassen und Auszuwerten. Diese können auch als Umgebungsqualitätswerte beschrieben sein und als Zeitreihe in der Cloud 17 abgelegt sein. Die Person 16 ist somit in der Lage, einen Schlafqualitätswert in Abhängigkeit eines Umgebungsqualitätswertes vergleichen zu können und eigene Rückschlüsse daraus ziehen zu können. Der Person 16 ist also die Möglichkeit gegeben, die Feinheiten zur Verbesserung des eigenen Schlafes selbst festlegen und bestimmen sowie selber erkennen zu können. Beispielsweise kann die Person 16 die optimale Umgebungstemperatur, die optimale Luftfeuchte, etc. selbst bestimmen, sowie die jeweilige Neigungsverstellung einzelner Stützflächen der Matratze oder des Bettes 1. Derartige Informationen zur Neigung oder Verstellhöhe einzelner Stützflächen des Bettes 1 bieten als bettspezifische Parameter ebenfalls wichtige Eingangsgrößen zur Bestimmung und Festlegung eines optimalen Schlafes und können ebenfalls in Zeitreihen und in einer Cloud 17 abgelegt sein.

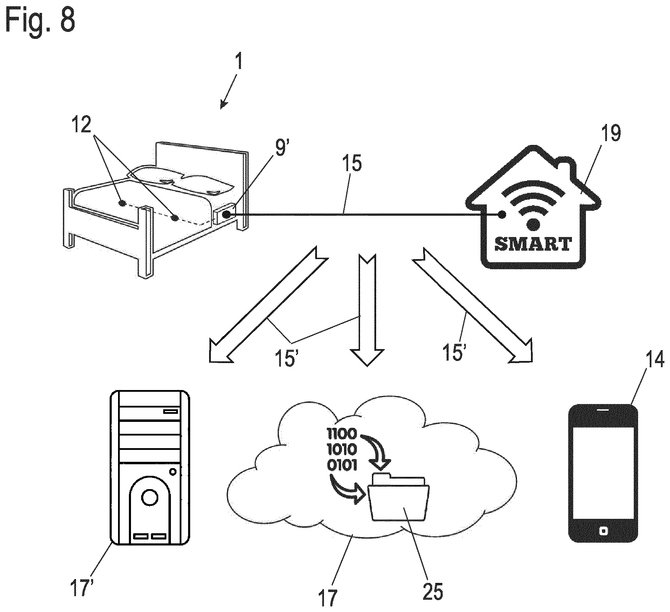

Umweltbezogene Parameter können von der Gebäudetechnik 19 bereit gestellt werden. Die Art und Weise der Bereitstellung sowie der Übertragung und Kommunikation zwischen der Auswerteeinheit 9', der Steuereinrichtung 9 und der Gebäudetechnik 19 ist durch Figur 8 näher illustriert.

Bettspezifische Parameter können von der Steuereinrichtung 9 bzw. der Auswerteeinheit 9' zur Verfügung gestellt werden und in Datenform auf diese übertragen werden, sowie als Informationswert oder als Zeitreihe ebenfalls Bestandteil der in der Cloud 17 abgelegten Daten 25 sein.

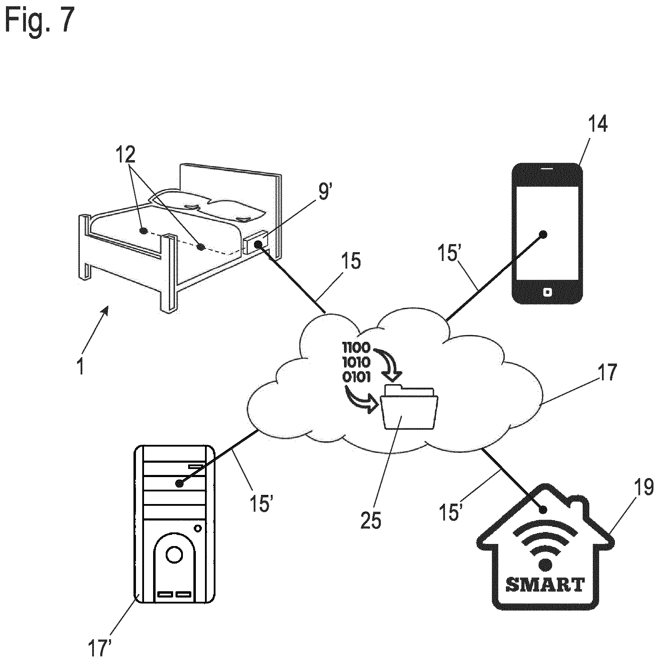

Fig. 7 zeigt ein weiteres Ausführungsbeispiel eines Systems vergleichbar mit dem in Fig. 5 und 6 gezeigten. Wiederum wird auf die Beschreibung zu den Figuren 5 und 6 verwiesen. Im Unterschied zu beispielsweise dem System der Fig. 6 ist bei dem der Fig. 7 eine Datenübertragung von der Auswerteeinheit 9' unmittelbar nur zur Cloud 17 vorgesehen. Weitere Geräte wie das Mobilgerät 14 und die Gebäudetechnik 19 greifen auf dem Weg der indirekten Datenübertragung 15' auf die in der Cloud 17 gespeicherten Daten 25 oder weitere dort hinterlegte Daten zu, wobei wiederum Zugriffsbeschränkungen auf nur bestimmte Teile der Daten vorgesehen sein können. In einer alternativen Ausgestaltung kann zusätzlich der Zugriff eines Servicecenters auf die in der Cloud 17 gespeicherten Daten vorgesehen sein.

Weiteren Zugriff auf die Daten 25 in der Cloud hat beim dargestellten Ausführungsbeispiel ein Rechendienst 17'. Der Rechendienst 17' deutet an, dass eine Weiterverarbeitung und Auswertung der Daten innerhalb der Cloud 17 durch eine Rechenleistung, die im Zusammenhang mit der Cloud 17 bereit gestellt wird, erfolgen kann.

Fig. 8 zeigt ein weiteres System zur Erfassung von physiologischen Parametern in gleicher Weise wie die Figuren 5 bis 7. Wiederum wird auf die Ausführung zu den vorherigen Ausführungsbeispielen verwiesen. Im Unterschied zu den bisherigen Beispielen steht bei diesem Beispiel die Verbindung zwischen der Auswerteeinheit 9' und der Gebäudetechnik 19 im Vordergrund. Nur zwischen diesen beiden besteht eine unmittelbare Datenverbindung. Darüber hinaus werden über indirekte Datenverbindungen 15' die von der Auswerteeinheit 9' und/oder der Gebäudetechnik 19 erfassten Daten abgegriffen und an die Cloud 17, den damit verbundenen Rechendienst 17' und/oder das Mobilgerät 14 übertragen.

Die im Zusammenhang mit den Figuren 1 bis 8 beschriebenen Datenübertragungen 15, 15' laufen innerhalb eines Netzwerkes ab. Das Netzwerk kann über genormte und/oder proprietäre Schnittstellen und Übertragungsstrecken verfügen. Während die direkte Datenverbindung gemäß des Beispiels nach Fig. 8 proprietärer Natur ist, sind andere Datenübertragungen 15' mittels genormter Übertragungsstrecke oder Schnittstelle ausgebildet, beispielsweise als LAN, WLAN, Bluetooth oder ZigBee.

Ist es erforderlich, dass Daten von einer Schnittstelle bzw. Übertragungsstrecke auf eine andere übertragen werden, können Modems oder Signalkonverter herangezogen werden, sodass beispielsweise ein Bluetooth-Gerät wie das Mobilgerät 14 seine Daten mit einem ZigBee-Gerät austauschen kann.

Da das Bett 1 mit seinen Komponenten wie der Steuereinrichtung 9 oder der Auswerteeinheit 9' ebenfalls Bestandteil eines solchen Netzwerkes ist, ist es möglich, Daten zwischen der Auswerteeinheit 9', der Gebäudetechnik 1, dem Mobilgerät 14, weiterer Einrichtungen einer Wohnung wie Fernseh- und Multimediageräte gebündelt einer Anzahl Steuerprogramme bereit zu stellen. Das jeweilige Steuerprogramm ist in der jeweiligen Einrichtung ausführbar.

Somit ist ein erstes Steuerprogramm der Gebäudetechnik 19 denkbar, welches in Abhängigkeit der Uhrzeit ein Verhaltensmuster der Person 16 erkennt und dieses als Prozedur "Zubettgehen" klassifiziert. Sodann erfolgt eine Übergabe von Informationen an die Steuereinrichtung 9 des Bettes 1, wobei eine nicht näher dargestellte Matratzenheizung durch die Steuereinrichtung 9 eingeschaltet wird. Das Aufheizen der Matratze kann zudem in Abhängigkeit der Zimmertemperatur erfolgen. Die Zimmertemperatur kann dabei durch einen Sensor gemessen werden, der der Gebäudetechnik 19 zugeordnet ist. Ein Wert der Zimmertemperatur kann über die Datenübertragungen 15' an die Steuereinrichtung 9 oder die Auswerteeinheit 9' übermittelt werden.

Ein anderes Steuerprogramm der Steuereinrichtung 9 und/oder der Auswerteeinheit 9' erfasst die Anwesenheit der Person 16 im Bett unter Zuhilfenahme des Signals der jeweiligen Sensoren 12 wie eingangs näher beschrieben. Ferner können Einschlafhilfen in Form von Routinen und Unterprogrammen vorgesehen sein, wobei das jeweilige Unterprogramm in Abhängigkeit der jeweiligen Schlafphase gesteuert wird. Ein erstes Unterprogramm kann das Licht leicht gedimmt halten. Ein weiteres Unterprogramm kann ein besonderes Lichtprogramm mit wohlfühlenden Farbverläufen steuern. Ein weiteres Unterprogramm kann das Aussenden von Entspannungsmelodien oder von Schlafmusik bewirken. Ein weiteres Unterprogramm, welches zu einer Schlafphase des regulären Schlafes gestartet wird, kann das Umgebungslicht und/oder die weiteren Einschlafhilfen oder sogar auch Multimediageräte wie Fernseher abschalten.

Das vorgenannte Bett 1 ist im Sinne dieser Anmeldung und Erfindung als Schlaf- und Ruhemöbel zu verstehen und soll beispielhaft für alle Arten von Schlaf- und Ruhemöbel genannt sein. Andere Ausführungen von Schlaf- und Ruhemöbeln sind Sessel oder Sofas. Insbesondere weisen Sessel zumindest ein bewegliches Rückenteil, ein bewegliches Beinteil und ein zwischen dem Rücken- und dem Beinteil angeordnetes Sitzteil auf, wobei die beweglichen Teile den Benutzer in eine nahezu liegende und schlafähnliche Position bringen können, weshalb damit die Analogie zu einem Bett 1 gegeben ist. Dieses gilt auch für Sofas, bei denen sich zumindest das Rückenteil derart bewegen lässt, dass das Sofa als Schlaf- oder Ruhemöbel dient.

### Bezugszeichenliste

- 1: Bett
- 2: Grundelement
- 3: Stützelement
- 4: Rückenteil
- 5: Beinteil
- 6: Bewegungsbeschlag
- 7, 8: Verstellantrieb
- 9: Steuereinrichtung
- 10: Handbedienung
- 11: Kabel
- 12: Sensor
- 13: Sensorkabel
- 14: Mobilgerät
- 15: direkte Datenübertragung
- 15': indirekte Datenübertragung
- 16: Person
- 17: Cloud
- 17': Rechendienst
- 18: Servicecenter
- 19: Gebäudetechnik

- 20: Signal
- 21: Herzschlag-Peak
- 22: einhüllende Flanke
- 23: ansteigende Flanke (Einatmung)
- 24: abfallende Flanke (Ausatmung)
- 25: Daten
- 26: erste Zeitreihe (Pulsfrequenz)
- 27: zweite Zeitreihe (Atemfrequenz)
- 28: dritte Zeitreihe (Bewegungsverhalten)
- 29: vierte Zeitreihe (Schnarchverhalten)

- M: Matratze
- P: physiologischer Parameter

## Patentansprüche

1. Anordnung eines Schlaf- oder Ruhemöbels und einer externen Komponente, wobei das Schlaf- oder Ruhemöbel mindestens einen Sensor (12) zur Erfassung von Vibrationen, Bewegungen und/oder Schall aufweist und eine mit diesem verbundene lokale Auswerteeinheit (9'), die zur Verarbeitung und Auswertung der Signale des mindestens einen Sensors (12) mit einer ersten Wiederholfrequenz und zur Erfassung von physiologischen Parametern (P) einer das Schlaf- oder Ruhemöbel benutzenden Person eingerichtet ist, und die zur Übertragung Daten (25), die die erfassten physiologischen Parameter (P) beinhalten, an mindestens ein Mobilgerät (14) als externe Komponente eingerichtet ist, wobei die lokale Auswerteeinheit (9') zur Übertragung der Daten oder zum Datenaustausch mit einer Steuereinrichtung (9) eines elektromotorischen Möbelantriebs eingerichtet ist, und wobei die lokale Auswerteeinheit (9') zur Übertragung der Daten an oder zum Datenaustausch mit dem Mobilgerät (14) eingerichtet ist, um Auswerte-, Darstellungs- und Kommunikationsmöglichkeiten des Mobilgeräts (14) nutzen zu können, **dadurch gekennzeichnet, dass** die lokale Auswerteeinheit (9') zum Analysieren der Sensordaten (20) und Ermitteln eines Schnarchverhaltens mindestens einer Person als physiologischen Parameter (P) mit einer zweiten Wiederholfrequenz, die kleiner ist als die erste Wiederholfrequenz, eingerichtet ist, und die lokale Auswerteeinheit (9') dazu eingerichtet ist, die physiologischen Parameter (P) in Form von Zeitverläufen (26, 27, 28, 29) zwischenzuspeichern, bevor sie an die externe Komponente übertragen werden, wobei in der lokalen Auswerteeinheit (9') Schlafzustände und Zeitanteile der Schlafzustände an einer Schlafperiode aus den Zeitverläufen (26, 27, 28, 29) extrahiert werden und ebenfalls an die externe Komponente übertragen werden.

2. Anordnung nach Anspruch 1, bei dem die Auswerteeinheit (9') zur Übertragung der Daten an oder zum Datenaustausch mit einem externen Massenspeicher als externe Komponente eingerichtet ist.

3. Anordnung nach Anspruch 2, wobei der externe Massenspeicher eine Cloud (17) ist.

4. Anordnung nach einem der Ansprüche 1 bis 3, bei dem die Auswerteeinheit (9') zur Übertragung der Daten an oder zum Datenaustausch mit einer Komponente einer Gebäudeautomatisierungsanlage (19) eingerichtet ist.

5. Anordnung nach einem der Ansprüche 1 bis 4, bei dem die Auswerteeinheit (9') einen Filter, insbesondere einen Tief- oder Bandpassfilter zur Signalverarbeitung aufweist.

6. Anordnung nach einem der Ansprüche 1 bis 5, bei dem die Auswerteeinheit (9') einen Speicher zum Zwischenspeichern eines Zeitverlaufs (26, 27, 28, 29) der physiologischen Parameter (P) aufweist.

7. Anordnung nach einem der Ansprüche 1 bis 6, bei dem die Auswerteeinheit (9') eine Übertragungseinheit zur drahtlosen Datenübertragung, insbesondere über eine WLAN- oder Bluetooth-Übertragungsstrecke, aufweist.

## Claims

1. Arrangement of a piece of sleeping or reclining furniture and an external component, wherein the piece of sleeping or reclining furniture has at least one sensor (12) for detecting vibrations, movements and/or sound and a local analyzing unit (9') connected thereto, which is set up to process and evaluate the signals of the at least one sensor (12) with a first repetition frequency and for detecting physiological parameters (P) of a person using the sleeping or reclining furniture, and which is designed to transmit data (25) containing the detected physiological parameters (P) to at least one mobile device (14) as an external component, wherein the local analyzing unit (9') is designed to transmit the data or to exchange data with a control unit (9) of an electric motor-driven furniture drive, and wherein the local analyzing unit (9') is designed to transmit the data to or to exchange data with the mobile device (14) in order to be able to use the evaluation, display and communication options of the mobile device (14), **characterized in that**
the local analyzing unit (9') is designed to analyze the signal (20) and determine the snoring behavior of at least one person as a physiological parameter (P) with a second repetition frequency that is lower than the first repetition frequency, and the local analyzing unit (9') is designed to temporarily store the physiological parameters (P) in the form of time series (26, 27, 28, 29) before they are transmitted to the external component, wherein sleep states and time proportions of the sleep states in a sleep period are extracted from the time series (26, 27, 28, 29) in the local analyzing unit (9') and are also transmitted to the external component.

2. Arrangement according to claim 1, wherein the analyzing unit (9') is set up as an external component for transmitting the data to or for exchanging data with an external mass storage device.

3. Arrangement according to claim 2, wherein the external mass storage device is a cloud (17).

4. Arrangement according to one of claims 1 to 3, wherein the analyzing unit (9') is designed to transmit the data to or exchange data with a component of a building technology (19).

5. Arrangement according to one of claims 1 to 4, wherein the analyzing unit (9') has a filter, in particular a low-pass or bandpass filter, for signal processing.

6. Arrangement according to one of claims 1 to 5, wherein the analyzing unit (9') has a memory for temporarily storing a time series (26, 27, 28, 29) of the physiological parameters (P).

7. Arrangement according to one of claims 1 to 6, wherein the analyzing unit (9') comprises a transmission unit for wireless data transmission, in particular via a WLAN or Bluetooth transmission link.

## Revendications

1. Agencement d'un meuble de couchage ou de repos avec un composant externe, dans lequel le meuble de couchage ou de repos comporte au moins un capteur (12) pour la détection de vibrations, de mouvements et/ou de sons et une unité d'analyse locale (9') reliée à celui-ci, qui est agencée pour traiter et analyser des signaux de l'au moins un capteur (12) à une première fréquence de répétition et pour acquérir des paramètres physiologiques (P) d'une personne utilisant le meuble de couchage ou de repos et qui est agencée pour transmettre des données (25) contenant les paramètres physiologiques (P) acquis à au moins un terminal mobile (14) formant le composant externe, dans lequel l'unité d'analyse locale (9') est agencée pour transmettre les données ou pour l'échange de données avec une installation de commande (9) d'un entraînement de meuble à moteur électrique, et dans lequel l'unité d'analyse locale (9') est agencée pour transmettre des données ou pour l'échange de données avec le terminal mobile (14) afin de pouvoir utiliser les possibilités d'analyse, de visualisation et de communication du terminal mobile (14), **caractérisé en ce que** l'unité d'analyse locale (9') est agencée pour analyser les données de capteur (20) et déterminer un comportement de ronflement d'au moins une personne sous forme de paramètres physiologiques (P) avec une deuxième fréquence de répétition qui est plus basse que la première fréquence de répétition, et l'unité d'analyse locale (9') est agencée pour stocker les paramètres physiologiques (P) sous forme de courbes dans le temps (26, 27, 28, 29) avant qu'ils soient transmis au composant externe, des états de sommeil et des parts de temps des états de sommeil sur une période de sommeil étant extraits de courbes dans le temps (26, 27, 28, 29) dans l'unité d'analyse locale (9') et également transmis au composant externe.

2. Agencement selon la revendication 1, dans lequel l'unité d'analyse locale (9') est agencée pour transmettre les données ou pour l'échange de données avec une mémoire de masse externe constituant le composant externe.

3. Agencement selon la revendication 2, dans lequel la mémoire de masse externe est un nuage (17).

4. Agencement selon l'une des revendications 1 à 3, dans lequel l'unité d'analyse locale (9') est agencée pour transmettre les données ou échanger les données avec une composante d'une installation d'automatisation de bâtiment (19).

5. Agencement selon l'une des revendications 1 à 4, dans lequel l'unité d'analyse locale (9') comporte un filtre, en particulier un filtre passe-bas ou passe-bande pour le traitement des signaux.

6. Agencement selon l'une des revendications 1 à 5, dans lequel l'unité d'analyse locale (9') comporte une mémoire pour le stockage d'une courbe dans le temps (26, 27, 28, 29) des paramètres physiologiques (P).

7. Agencement selon l'une des revendications 1 à 6, dans lequel l'unité d'analyse locale (9') comporte une unité de transmission pour la transmission sans fil de données, en particulier via un trajet de transmission par réseau local sans fil ou par Bluetooth.
